# EUROPEAN PATENT APPLICATION

(11) **EP 4 410 822 A1**
(43) Date of publication of application: **07.08.2024**
(21) Application number: 22889984.5
(22) Date of filing: 02.11.2022
(51) Int. Cl.: C07K 14/00, C07K 1/02, C08G 69/48

(54) **ESTERIFIED PROTEIN AND PRODUCTION METHOD THEREOF**

(30) Priority: 02.11.2021 JP 2021179774; 30.11.2021 JP 2021194264
(71) Applicant: Spiber Inc., Tsuruoka-shi, Yamagata 997-0052 (JP)
(72) Inventor: SAKATA Kazuki, Tsuruoka-shi, Yamagata 997-0052 (JP); SHIRAKAWA Seiji, Nagasaki-shi, Nagasaki 852-8521 (JP); KITAHARA Nao, Tsuruoka-shi, Yamagata 997-0052 (JP); NIWA Takahiro, Tsuruoka-shi, Yamagata 997-0052 (JP)
(74) Representative: Handley, Matthew Edward
(86) International application number: PCT/JP2022/041016
(87) International publication number: WO 2023/080164

(57) **Abstract**

The present invention provides a β-ketoester of a protein containing an amino acid having a hydroxyl group, wherein the hydroxyl group forms an ester bond with a group represented by Formula (1) wherein R represents a C₁₋₆ alkyl group or an optionally substituted aryl group.

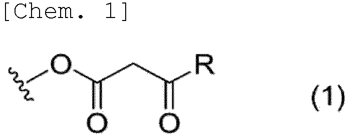

## Description

### Technical Field

The present invention relates to an esterified protein and a method for producing the same.

### Background Art

In recent years, due to the growing awareness of environmental conservation, studies on alternative substances for petroleum-derived materials have been conducted, and proteins have attracted attention in terms of strength and the like. For example, Patent Literature 1 discloses that a molded product obtained by compression-molding wool, cashmere fibers, down, and the like has high mechanical characteristics such as stress-strain characteristics. In addition, materialization of an artificial protein that can be industrially produced has been studied. In particular, application research as a molding material of artificial fibroin having characteristics of spider silk excellent in strength and elongation has been conducted (for example, Patent Literatures 1 to 7). Studies have also been made to chemically modify amino acid residues of artificial fibroin having spider silk characteristics (for example, Patent Literatures 8 and 9).

### Citation List

### Patent Literature

Patent Literature 1: JP 2018-150637 A
Patent Literature 2: JP 2018-178296 A
Patent Literature 3: JP 2019-131923 A
Patent Literature 4: JP 2020-97524 A
Patent Literature 5: JP 2020-97796 A
Patent Literature 6: WO 2017/188430 A
Patent Literature 7: WO 2017/188434 A
Patent Literature 8: WO 2021/187502 A
Patent Literature 9: WO 2021/187500 A
Patent Literature 10: JP 2012-21013 A

### Summary of Invention

### Technical Problem

The present inventors have studied production and purification of various artificial proteins, and found that when the protein has high hydrophilicity, there is a problem that the protein is dissolved in water and flows, whereas when the protein has high hydrophobicity, there is a problem that it is difficult to separate the protein from a highly lipid-soluble component such as a lipid. Such a problem of purification also causes a problem that it is difficult to widen the range of proteins that can be synthesized.

Therefore, an object of the present invention is to provide a protein derivative capable of introducing various functional groups by chemically modifying a protein after a purification step.

### Solution to Problem

The present inventors have found that various functional groups can be introduced by a nucleophilic reaction or an electrophilic reaction when a derivative obtained by β-ketoesterifying an amino acid residue having a hydroxyl group (for example, serine, threonine, tyrosine) contained in the protein is used.

The present invention provides the following [1] to [28] .

[1] A β-ketoester of a protein containing an amino acid having a hydroxyl group, wherein
   the hydroxyl group forms an ester bond with a group represented by Formula (1): 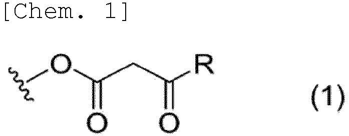
   wherein R represents a C₁₋₆ alkyl group or an optionally substituted aryl group.
[2] The β-ketoester of the protein according to [1], wherein the protein is a hydrophobic protein.
[3] The β-ketoester of the protein according to [2], wherein the hydrophobic protein includes a protein having an average hydropathy index (HI) of -1 or more.
[4] The β-ketoester of the protein according to any one of [1] to [3], wherein the protein is an artificial protein.
[5] A method for producing the β-ketoester of the protein according to [1],
   the method including a step of mixing the protein containing an amino acid having a hydroxyl group and a compound represented by Formula (2a) or (2b) in a solvent, and performing stirring while heating: 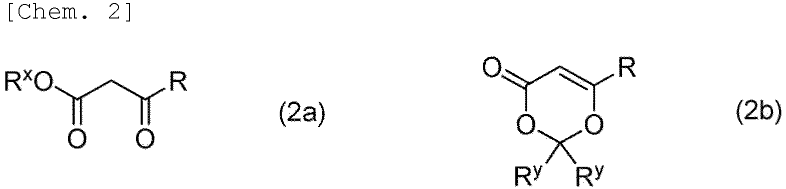
   wherein R represents a C₁₋₆ alkyl group or an optionally substituted aryl group, and R^{x} and R^{y} each independently represent a C₁₋₆ alkyl group or an optionally substituted aryl group.
[6] The method for producing the β-ketoester of the protein according to [5], wherein the protein is a hydrophobic protein.
[7] The method for producing the β-ketoester of the protein according to [6], wherein the hydrophobic protein includes a protein having an average hydropathy index (HI) of 0 or more.
[8] The method for producing the β-ketoester of the protein according to any one of [5] to [7], wherein the protein includes an artificial protein.
[9] A method for producing a chemically modified protein having a group represented by Formula (3),
   the method including a step of mixing the β-ketoester of the protein according to [1] and a compound represented by Formula (4) in a solvent: 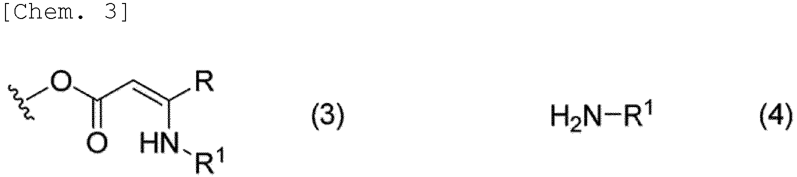
   wherein R represents a C₁₋₆ alkyl group or an optionally substituted aryl group, and R¹ represents an organic group.
[10] A chemically modified protein having a group represented by Formula (3): 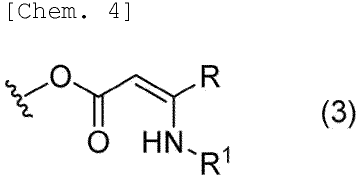 wherein R represents a C₁₋₆ alkyl group or an optionally substituted aryl group, and R¹ represents an organic group.
[11] A method for producing a chemically modified protein having a group represented by Formula (5),
   the method including a step of mixing the β-ketoester of the protein according to [1] and a compound represented by Formula (6) in a solvent: 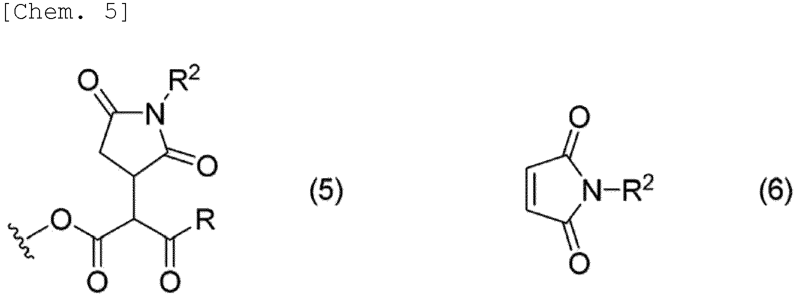
   wherein R represents a C₁₋₆ alkyl group or an optionally substituted aryl group, and R² represents an organic group.
[12] A chemically modified protein having a group represented by Formula (5): 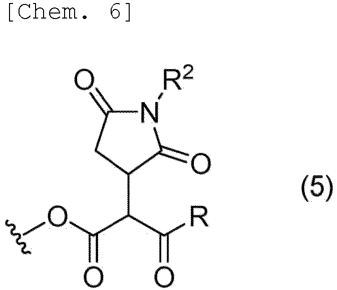 wherein R represents a C₁₋₆ alkyl group or an optionally substituted aryl group, and R² represents an organic group.
[13] A method for producing a chemically modified protein having a group represented by Formula (7),
   the method including a step of mixing the β-ketoester of the protein according to [1] and a compound represented by Formula (8) in a solvent: 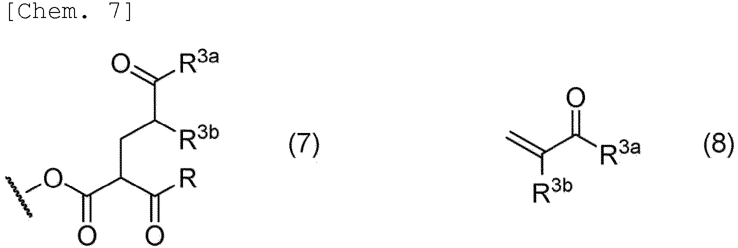
   wherein R represents a C₁₋₆ alkyl group or an optionally substituted aryl group, and R^{3a} and R^{3b} each independently represent an organic group.
[14] A chemically modified protein having a group represented by Formula (7): 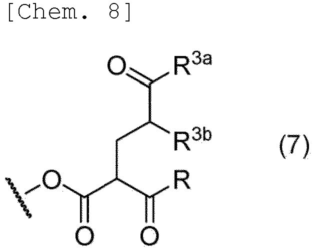 wherein R represents a C₁₋₆ alkyl group or an optionally substituted aryl group, and R^{3a} and R^{3b} each independently represent an organic group.
[15] A method for producing a chemically modified protein having a group represented by Formula (9),
   the method including a step of mixing the β-ketoester of the protein according to [1] and a compound represented by Formula (10) in a solvent: 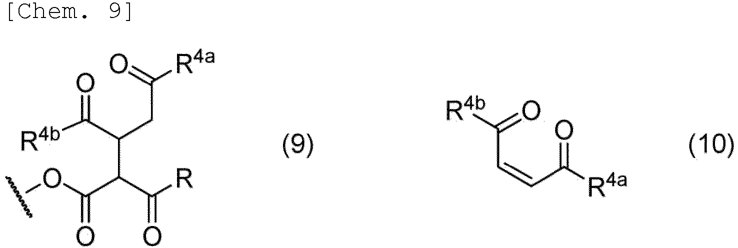
   wherein R represents a C₁₋₆ alkyl group or an optionally substituted aryl group, and R^{4a} and R^{4b} each independently represent an organic group.
[15] A chemically modified protein having a group represented by Formula (9): 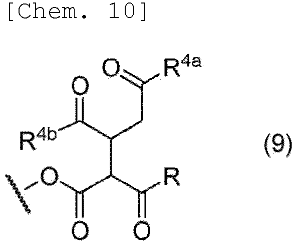 wherein R represents a C₁₋₆ alkyl group or an optionally substituted aryl group, and R^{4a} and R^{4b} each independently represent an organic group.
[16] A method for producing a chemically modified protein having a group represented by Formula (11),
   the method including a step of mixing the β-ketoester of the protein according to [1] and a compound represented by Formula (12) in a solvent: 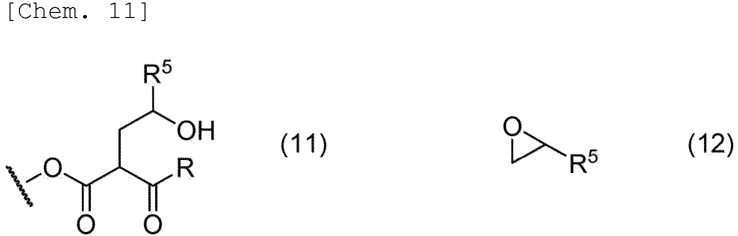
   wherein R represents a C₁₋₆ alkyl group or an optionally substituted aryl group, and R⁵ represents an organic group.
[17] A chemically modified protein having a group represented by Formula (11): 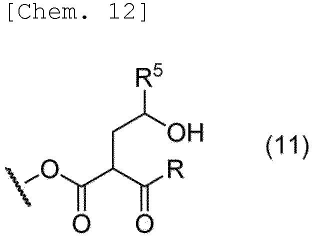 wherein R represents a C₁₋₆ alkyl group or an optionally substituted aryl group, and R⁵ represents an organic group.
[18] A method for producing a chemically modified protein having a group represented by Formula (13),
   the method including a step of mixing the β-ketoester of the protein according to [1] and a compound represented by Formula (14) in a solvent: 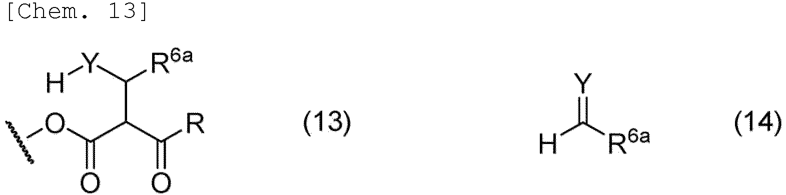
   wherein R represents a C₁₋₆ alkyl group or an optionally substituted aryl group, R^{6a} represents an organic group, Y represents an oxygen atom or NR^{6b}, and R^{6b} represents an organic group.
[19] A chemically modified protein having a group represented by Formula (13): 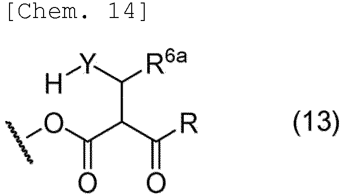 wherein R represents a C₁₋₆ alkyl group or an optionally substituted aryl group, R^{6a} represents an organic group, Y represents an oxygen atom or NR^{6b}, and R^{6b} represents an organic group.
[20] A method for producing a chemically modified protein having a group represented by Formula (15),
   the method including a step of mixing the β-ketoester of the protein according to [1] and a compound represented by Formula (16) in a solvent: 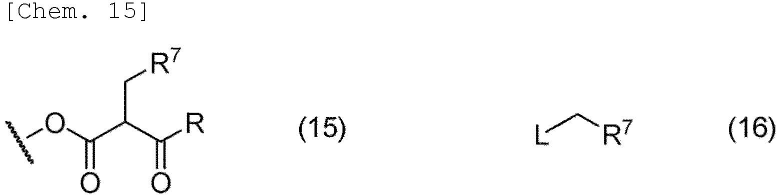
   wherein R represents a C₁₋₆ alkyl group or an optionally substituted aryl group, R⁷ represents an organic group, and L represents a leaving group.
[21] A chemically modified protein having a group represented by Formula (15): 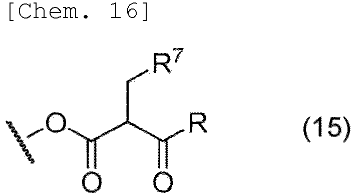 wherein R represents a C₁₋₆ alkyl group or an optionally substituted aryl group, R⁷ represents an organic group, and L represents a leaving group.
[22] A method for producing a chemically modified protein having a group represented by Formula (17),
   the method including a step of mixing the β-ketoester of the protein according to [1] and a compound represented by Formula (18) in a solvent: 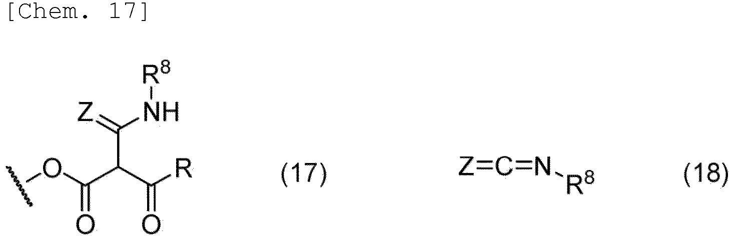
   wherein R represents a C₁₋₆ alkyl group or an optionally substituted aryl group, R⁸ represents an organic group, and Z represents an oxygen atom or a sulfur atom.
[23] A chemically modified protein having a group represented by Formula (17): 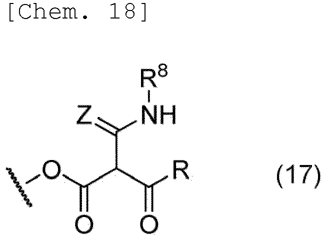 wherein R represents a C₁₋₆ alkyl group or an optionally substituted aryl group, R⁸ represents an organic group, and Z represents an oxygen atom or a sulfur atom.

### Advantageous Effects of Invention

In general, activated carboxylic acid derivatives such as acid halides and acid anhydrides are used as conditions for esterifying a hydroxyl group. However, proteins have low solubility in a solvent, and are treated with formic acid or dimethyl sulfoxide (DMSO) as a solvent. When formic acid is used as a solvent, a protein having a hydroxyl group and formic acid are often esterified. In addition, when DMSO is used as a solvent and an activated carboxylic acid derivative is added, sulfoxide oxygen of DMSO is activated to proceed a Swern oxidation type oxidation reaction and oxidize a hydroxyl group in a protein to an aldehyde, further causing an unexpected side reaction. Patent Literature 10 discloses a method for reacting a dipeptide with a diketene to perform amidation, but a simple method for esterifying a hydroxyl group of a protein is not known.

According to the present invention, a hydroxyl group in a protein can be esterified by a simple method by using a tert-butyl ester represented by Formula (2) as an acylating agent. Furthermore, the obtained β-ketoester has excellent reactivity, and thus various functional groups can be introduced into the β-ketoester by reacting the β-ketoester with an electrophile or a nucleophile. Specifically, it is possible to impart a fluorescent labeling function, hydrophobicity, solubility in a solvent, thermal stability (for example, increase or decrease in glass transition temperature Tg), and the like to a protein. The degree of such functions can also be adjusted by adjusting the number of functional groups to be introduced. Therefore, it becomes possible to adjust the physical properties of the protein, and it possible to provide a chemically modified protein having similar physical properties as a substitute for a protein having physical properties that make culture purification difficult. According to the invention of the present application, the usefulness of existing proteins can be further enhanced.

### Brief Description of Drawings

Fig. 1 shows ¹H-NMR spectra of PRT966 and acetoacetylated PRT966.
Fig. 2 shows ¹H-NMR spectra of PRT587 and acetoacetylated PRT587.
Fig. 3 shows ¹H-NMR spectra of silk and acetoacetylated silk.
Fig. 4 shows ¹H-NMR spectra of casein and acetoacetylated casein.
Fig. 5 shows ¹H-NMR spectra of collagen and acetoacetylated collagen.
Fig. 6 shows IR spectra of PRT966 and acetoacetylated PRT966.
Fig. 7 shows IR spectra of PRT587 and acetoacetylated PRT587.
Fig. 8 shows IR spectra of silk and acetoacetylated silk.
Fig. 9 shows IR spectra of casein and acetoacetylated casein.
Fig. 10 shows IR spectra of collagen and acetoacetylated collagen.
Fig. 11(a) shows a GPC chromatogram of PRT966 and acetoacetylated PRT966, Fig. 11(b) is a GPC chromatogram of PRT587 and acetoacetylated PRT587, and Fig. 11(c) is a GPC chromatogram of silk and acetoacetylated silk.
Fig. 12(a) is a GPC chromatogram of casein and acetoacetylated casein, and Fig. 12(b) is a GPC chromatogram of collagen and acetoacetylated collagen.
Fig. 13 is a photograph of a gel showing the results of SDS-PAGE of each protein.
Fig. 14 is a graph showing the results of fluorescence analysis of an acetoacetylated protein to which sodium 5-(2-aminoethylamino)-1-naphthalenesulfonate is added.
Fig. 15 is a ¹H-NMR spectrum of an acetoacetylated protein to which benzylamine is added.
Fig. 16 is a ¹H-NMR spectrum of an acetoacetylated protein to which octylamine is added.
Fig. 17 is a ¹H-NMR spectrum of an acetoacetylated protein to which furfurylamine is added.
Fig. 18 is a graph showing the results of fluorescence analysis of an acetoacetylated protein to which N-(1-pyrenyl)-maleimide is added.
Fig. 19 is a ¹H-NMR spectrum of a benzoylacetylated protein to which tert-butyl benzoylacetate is added.
Fig. 20 is photographs showing the state of a material after solidification in the case of using DMSO dope solutions A to C.

### Description of Embodiments

Hereinafter, the present invention will be described in more detail.

A first embodiment of the present invention is a β-ketoester of a protein containing an amino acid having a hydroxyl group, wherein the hydroxyl group forms an ester bond with a group represented by Formula (1): 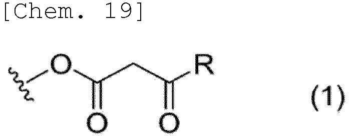 wherein R represents a C₁₋₆ alkyl group or an optionally substituted aryl group.

In Formula (1), R is a C₁₋₆ alkyl group or an optionally substituted aryl group. Examples of the C₁₋₆ alkyl group include a methyl group, an ethyl group, a 1-propyl group, a 2-propyl group, a 1-butyl group, a 2-butyl group, a 1-pentyl group, a 2-pentyl group, a 3-pentyl group, a neopentyl group, a 1-hexyl group, a 2-hexyl group, and a 3-hexyl group. Examples of the aryl group include aromatic hydrocarbon ring groups such as a phenyl group and a naphthalene group, and heterocyclic groups such as a pyridyl group, a pyrimidinyl group, a pyrazinyl group, a furanyl group, a thiophenyl group, a pyrrole group, a quinolinyl group, and an isoquinyl group. The aryl group may be further substituted with one or more groups selected from a C₁₋₆ alkyl group, a C₁₋₆ alkoxy group, a halogen atom, a sulfo group, and a carboxy group. Examples of the substituted aryl group include an m-methylphenyl group and a p-methoxyphenyl group.

The protein containing an amino acid having a hydroxyl group may be any protein as long as it contains at least one amino acid having a hydroxyl group, and may be a natural protein or an artificial protein. Examples of the artificial protein include any protein that can be produced on an industrial scale. Examples of an amino acid residue having a hydroxyl group include a serine (S) residue, a threonine (T) residue, and a tyrosine (Y) residue. The hydroxyl group is acylated to form a β-ketoester structure represented by Formula (1). That is, the oxa group of the β-ketoester skeleton is derived from the hydroxyl group of the amino acid residue.

The β-ketoester of the protein according to the present embodiment only needs to contain at least one group represented by Formula (1), and may contain two or more groups.

Examples of the protein include a protein that can be used for industrial purposes, a protein that can be used for medical purposes, and a structural protein. The phrase "can be used for industrial purposes" means, for example, that it can be used for various general-purpose materials used indoors or outdoors. Specific examples of the protein that can be used for medical purposes include an enzyme, a regulatory protein, a receptor, a peptide hormone, a cytokine, a membrane or transport protein, an antigen used for vaccination, a vaccine, an antigen-binding protein, an immunostimulatory protein, an allergen, and a full length antibody or an antibody fragment or a derivative thereof. Examples of the protein that can be used for industrial purposes include structural proteins. The structural protein may be a protein related to a structure of a living body, a protein included in a structure created by a living body, or a protein derived from these proteins. The structural protein refers to a protein that is self-aggregated under certain conditions to form a structure such as a fiber, a film, a resin, a gel, a micelle, or a nanoparticle. Specific examples thereof include spider silk, fibroin such as silkworm silk, keratin, collagen, elastin, and resilin, and proteins derived therefrom. As the artificial protein to be used, modified fibroin is preferable, and artificial spider silk fibroin (artificial modified spider silk fibroin) is more preferable.

The artificial protein includes a recombinant protein and a synthetic protein. That is, in the present specification, the "artificial protein" means a protein artificially produced. The artificial protein may be a protein having an amino acid sequence different from an amino acid sequence of a naturally derived protein, or may include a protein having an amino acid sequence identical to an amino acid sequence of a naturally derived protein. The "artificial protein" may be a protein obtained by using an amino acid sequence of a naturally derived protein as it is, a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence). Here, the amino acid sequence of the artificial protein can be freely designed unlike the natural protein. Therefore, when such an artificial protein is used as a chemically modified artificial protein, it is possible to arbitrarily control the functions, properties, physical properties, and the like of the molding material and the molded body by appropriately designing the amino acid sequence of the artificial protein. Since a uniform molecular design is always possible, a protein that has high homology with a target protein and is suitable for a purpose can be stably obtained. As a result, it is advantageous to stabilize the quality of a target chemically modified artificial protein, and eventually, a molding material or a molded body obtained using the chemically modified artificial protein.

The protein according to the present embodiment may have 50 or more amino acid residues. The number of amino acid residues may be, for example, 100 or more or 150 or more, or 200 or more or 250 or more, and preferably 300 or more, 350 or more, 400 or more, 450 or more, or 500 or more. The number of amino acid residues is, for example, 5,000 or less, 4,500 or less, 4,000 or less, 3,500 or less, 3,000 or less, 2,500 or less, 2,000 or less, 1,500 or less, or 1,000 or less. As the number of amino acid residues is smaller, the solubility in the solvent tends to increase. Therefore, when the number of amino acid residues of the protein according to the present embodiment is, for example, 5,000 or less or 2,500 or less, the protein is dissolved in a solvent and reacted with the compound represented by Formula (2a) or (2b), whereby the production efficiency of the β-ketoester of the target protein can be improved.

The molecular weight of the protein according to the present embodiment may be, for example, 1,000 or more, 2,000 or more, 3,000 or more, 4,000 or more, 5,000 or more, 6,000 or more, 7,000 or more, 8,000 or more, 9,000 or more, 10,000 or more, 20,000 or more, 30,000 or more, 40,000 or more, 50,000 or more, 60,000 or more, 70,000 or more, 80,000 or more, 90,000 or more, or 100,000 or more. Furthermore, the molecular weight of such a protein may be 400,000 or less, less than 360,000, 300,000 or less, or 200,000 or less. As the molecular weight of the protein is smaller, the solubility in the solvent tends to increase. Therefore, when the molecular weight of the protein according to the present embodiment is, for example for example, 200,000 or less or 100,000 or less, the protein is dissolved in a solvent and reacted with a compound represented by Formula (2a) or (2b), whereby the production efficiency of the β-ketoester of the target protein can be improved.

The protein according to the present embodiment preferably has a high proportion of amino acid residues such as glycine residues, alanine residues, and serine residues. This is because an amino acid having a smaller side chain is more likely to be hydrogen-bonded to obtain a molded body having higher strength. Since the alanine residues and the glycine residues are amino acids having side chains with a small bulk, the alanine residues and the glycine residues are arranged to face inward in a folding process during the polypeptide production and tend to have an α-helix structure or a β-sheet structure. Therefore, it is desirable that the proportion of amino acids such as a glycine residue, an alanine residue, and a serine residue is high. For example, the alanine residue content may be 5 to 40%, 6 to 40%, 7 to 40%, 8 to 40%, 9 to 40%, 10 to 40%, 11 to 40%, 12 to 40%, 13 to 40%, 14 to 40%, 15 to 40%, 18 to 40%, 20 to 40%, or 22 to 40%. For example, the glycine residue content may be 10 to 55%, 11% to 55%, 13% to 55%, 15% to 55%, 18% to 55%, 20% to 55%, 22% to 55%, 25% to 55%, 28% to 55%, or 30% to 55%.

Herein, the "alanine residue content" is a value represented by the following equation. Alanine residue content = (Number of alanine residue contained in polypeptide/Number of all amino acid residue of polypeptide) × 100 (%)

In addition, the glycine residue content, the serine residue content, the threonine residue content, the proline residue content, and the tyrosine residue content have the same meanings as those obtained by replacing the alanine residue with the glycine residue, the serine residue, the threonine residue, the proline residue, and the tyrosine residue, respectively, in the above equation.

From the viewpoint of improving processability, it is important to inhibit strong hydrogen bonding between molecules at the time of processing, and from this viewpoint, it is desirable that an amino acid having a large side chain or an amino acid having flexibility is uniformly contained in the entire sequence to a certain extent. Specifically, a motif containing a tyrosine residue, a threonine residue, or a proline residue may be repeated and inserted in a cycle. For example, the total content of the proline residues, the threonine residues, and the tyrosine residues in arbitrary 20 consecutive amino acid residues may be 5% or more, 10% or more, or 15% or more, and may be 50% or less, 40% or less, 30% or less, or 20% or less.

The protein according to the present embodiment is preferably a protein having a large total number of serine residues, threonine residues, and tyrosine residues. The total number of serine residues, threonine residues, and tyrosine residues may be more than 0% and 65% or less, and is preferably more than 0% and 60% or less, more than 0% and 55% or less, more than 0% and 50% or less, more than 0% and 45% or less, more than 0% and 39% or less, more than 0% and 35% or less, 5 to 65%, 5 to 60%, 5 to 55%, 5 to 50%, 5 to 45%, 5 to 39%, 5 to 35%, 9 to 65%, 9 to 60%, 9 to 55%, 9 to 50%, 9 to 45%, 9 to 39%, 9 to 35%, 13 to 65%, 13 to 60%, 13 to 55%, 13 to 50%, 13 to 45%, 13 to 39%, 13 to 35%, 15 to 65%, 15 to 60%, 15 to 55%, 15 to 50%, 15 to 45%, 15 to 39%, or 15 to 35%, based on the total number of residues constituting the protein. When the proportion of serine, threonine, and tyrosine is within the above range, solubility in an aprotic polar solvent (for example, dimethyl sulfoxide (DMSO), N,N-dimethylformamide (DMF), N,N-dimethylacetamide (DMAc), and N-methylpyrrolidone (NMP)) increases. In addition, the number of β-ketoesters to be introduced can be increased, so that the range in which the physical properties of the protein can be adjusted can be further widened.

In the protein according to the present embodiment, in order to obtain the above-described effect more reliably, the sum of the serine residue content, the threonine residue content, and the tyrosine residue content may be 4% or more, 4.5% or more, 5% or more, 5.5% or more, 6% or more, 6.5% or more, 7% or more, 8% or more, 100 or more, 12% or more, 15% or more, 18% or more, 20% or more, 25% or more, or 28% or more. The sum of the serine residue content, the threonine residue content, and the tyrosine residue content may be, for example, 35% or less, 34% or less, 33% or less, 32% or less, 31% or less, 30% or less, 28% or less, or 25% or less.

The proportion of total of serine residues, threonine residues, and tyrosine residues in silk, collagen, and sericin are 18.1%, 12%, and 60.5%, respectively.

In addition, the β-ketoester of the protein according to the present embodiment exhibits effects of excellent coagulability in the coagulation bath in the DMSO spinning system and reduction of the shrinkage rate in the fibrous state, as compared with the corresponding protein.

The protein according to the present embodiment may have a repetitive sequence. That is, the polypeptide according to the present embodiment may have a plurality of amino acid sequences (repetitive sequence units) having high sequence identity in the polypeptide. The number of amino acid residues of the repetitive sequence units is preferably 6 to 200. The sequence identity between the repetitive sequence units may be, for example, 80% or more, 85% or more, 90% or more, 95% or more, 96% or more, 97% or more, 98% or more, or 99% or more. The hydropathy index (average hydropathy index) of the repetitive sequence unit may be, for example, -1.0 or more, -0.9 or more, -0.8 or more, -0.7 or more, -0.6 or more, -0.5 or more, -0.4 or more, -0.3 or more, -0.2 or more, -0.1 or more, 0 or more, 0.1 or more, 0.2 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. It is to be noted that an upper limit of the hydrophobic degree of the repetitive sequence unit is not particularly limited, and may be, for example, 1.0 or less or 0.7 or less.

The protein according to the present embodiment may contain a domain sequence represented by Formula 1: [REP1-REP2]ₘ or Formula 2: [REP1-REP2]ₘ-REP1. REP1 represents an amino acid sequence consisting of 2 to 27 amino acid residues. REP1 is preferably an amino acid sequence consisting of 2 to 20 amino acid residues, and more preferably an amino acid sequence consisting of 2 to 16 amino acid residues. REP1 includes an amino acid sequence having mainly alanine residues, and the number of amino acid residues may be 2 to 27, and may be an integer of 2 to 20, 2 to 16, or 2 to 12. The ratio of the number of alanine residues to the total number of amino acid residues in REP1 only needs to be 40% or more, and may be 60% or more, 70% or more, 80% or more, 83% or more, 85% or more, 86% or more, 90% or more, 95% or more, or 100% (which means consisting of only of alanine residues). A plurality of REP1s present in the protein according to the present embodiment may be the same amino acid sequence or different amino acid sequences. "m" in Formulas 1 and 2 means the number of repetitions of the amino acid sequence represented by [REP1-REP2], and represents an integer of 8 to 300.

In a preferred embodiment of the first embodiment of the present invention, the hydroxyl group of the amino acid residue is a β-ketoester forming an ester bond with an acetoacetyl group or a benzoylacetyl group.

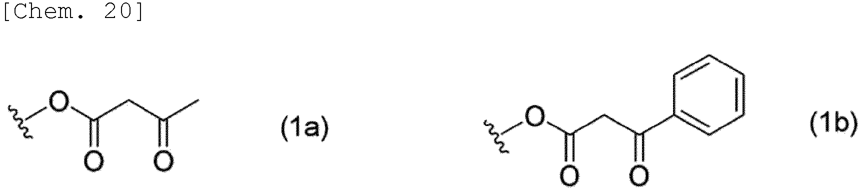

The protein according to the present embodiment is preferably a chemically modified hydrophobic protein. The chemically modified hydrophobic protein is obtained by chemically modifying a hydrophobic protein. The hydrophobic protein can be determined based on at least one of a hydropathy index (average hydropathy index) of an amino acid residue described later, solubility, a contact angle with water, and presence or absence of decomposition by hot water. In a preferred hydrophobic artificial protein, the hydropathy index can be, for example, -1.0 or more, -0.9 or more, -0.8 or more, -0.7 or more, -0.6 or more, -0.5 or more, -0.4 or more, -0.3 or more, -0.2 or more, -0.1 or more, 0 or more, 0.1 or more, 0.2 or more, 0.22 or more, 0.25 or more, 0.3 or more, 0.35 or more, 0.4 or more, 0.45 or more, 0.5 or more, 0.55 or more, or 0.6 or more, 0.65 or more, or 0.7 or more. The upper limit of the hydropathy index is not particularly limited, but may be, for example, 1.0 or less or 0.7 or less. In a preferred hydrophobic protein, the maximum concentration (solubility) when the hydrophobic protein is dissolved in a lithium bromide aqueous solution (concentration: 9 M) at 60°C may be, for example, 30 mass%, less than 25 mass%, less than 20 mass%, less than 15 mass%, less than 10 mass%, less than 5 mass%, or less than 1 mass%. A preferred hydrophobic protein may be completely insoluble in a lithium bromide aqueous solution (concentration: 9 M) at 60°C. Furthermore, in a preferred hydrophobic protein, the water contact angle (when water is dropped onto a membrane formed using an artificial protein, or after a lapse of a predetermined time after the water was dropped (for example, after a lapse of 5 seconds)) can be 55° or more, 60° or more, 65° or more, or 70° or more. In addition, a preferred hydrophobic protein has excellent hot water resistance. For example, a dispersion containing a protein and water and having a protein content of 5 mass% is prepared, and a protein that is not decomposed even when the dispersion is heat-treated at 100°C for 5 hours is determined to be the hydrophobic protein.

As the hydropathy index of an amino acid residue, a known index (Hydropathy index: Kyte J & Doolittle R (1982) "A simple method for displaying the hydropathic character of a protein", J. Mol. Biol., 157, pp. 105 to 132) is used. Specifically, a hydropathy index (hereinafter, also referred to as "HI") of each amino acid is indicated in the following Table 1.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Gln) | -3.5 |
| Threonine (Thr) | -0.7 | Lysine (Lys) | -3.9 |
| Serine (Ser) | -0.8 | Arginine (Arg) | -4.5 |

When the protein according to the present embodiment is a chemically modified hydrophobic protein obtained by chemically modifying the hydrophobic protein as described above, for example, improvement in water resistance can be expected. In addition, when such a chemically modified hydrophobic protein is used, for example, as a molding material, improvement in water resistance of a molded body produced using such a molding material can be desired. When such a molded body is used as a general-purpose material for industrial use, the extension of service life of the molded body can be expected based on water resistance. In addition, when a functional substance is bound to the chemically modified protein, the hydrophobicity or hydrophilicity of the entire conjugate of the functional substance and the chemically modified protein can be arbitrarily adjusted by controlling the hydrophobicity or hydrophilicity of the functional substance. When the protein has hydrophobicity, the entire conjugate can be shifted to the hydrophobic side as compared with the case where the protein has hydrophilicity. As a result, the hydrophobicity or hydrophilicity of the entire synthetic polymer can be controlled over a wider range.

### (Artificial structural protein)

In the present specification, the "artificial structural protein" means a structural protein artificially produced. The artificial structural protein may be any artificial structural protein into which a β-ketoester group can be introduced by chemical modification. The artificial structural protein may be a structural protein produced through microbial production using genetic recombination, also includes a structural protein whose amino acid sequence is improved from the viewpoint of moldability and productivity, and is not limited to a structural protein having a sequence of a naturally derived structural protein.

### (Artificial fibroin)

As the artificial structural protein, artificial fibroin is preferred. An example of the fibroin includes naturally derived fibroin. Examples of the naturally derived fibroin include fibroin produced by insects or spiders.

More specific examples of the naturally derived fibroin include fibroin having sequence information registered in NCBI GenBank. For example, it can be confirmed by extracting a sequence in which spidroin, ampullate, fibroin, "silk and polypeptide", or "silk and protein" is described as a keyword in DEFINITION from sequences including INV as DIVISION among pieces of sequence information registered in NCBI GenBank, a sequence in which a specific character string of product is described from CDS, or a sequence in which a character string specific to TISSUE TYPE is described from SOURCE.

The "artificial fibroin" herein means artificially produced fibroin (artificial fibroin). The artificial fibroin may be fibroin having an amino acid sequence different from or identical to an amino acid sequence of naturally derived fibroin.

The artificial fibroin may be a fibrous protein having a structure similar to that of naturally derived fibroin, or may be fibroin having a sequence similar to a repetitive sequence of naturally derived fibroin. The "sequence similar to a repetitive sequence of fibroin" may actually be a sequence of naturally derived fibroin, or may be a sequence similar thereto.

The "artificial fibroin" may be fibroin whose amino acid sequence is modified based on naturally derived fibroin (for example, fibroin whose amino acid sequence is modified by altering a cloned gene sequence of naturally derived fibroin) or fibroin obtained by artificially designing an amino acid sequence independently of naturally derived fibroin (for example, fibroin having a desired amino acid sequence by chemically synthesizing a nucleic acid encoding the designed amino acid sequence) as long as the artificial fibroin has an amino acid sequence specified in the present disclosure. Note that fibroin obtained by modifying an amino acid sequence of artificial fibroin is also included in artificial fibroin as long as the amino acid sequence thereof is different from the amino acid sequence of naturally derived fibroin. Examples of the artificial fibroin include artificial silk fibroin (fibroin obtained by modifying an amino acid sequence of a silk protein produced by silkworms) and artificial spider silk fibroin (fibroin obtained by modifying an amino acid sequence of a spider silk protein produced by spiders). The artificial fibroin preferably contains artificial spider silk fibroin as a molding material, and more preferably consists of artificial spider silk fibroin because it is relatively easily fibrillated and has a high fiber forming ability.

The artificial fibroin according to the present embodiment may be a protein having a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to any one or both of the N-terminal side and the C-terminal side of the domain sequence of the artificial fibroin. The N-terminal sequence and the C-terminal sequence are typically regions not containing repeats of amino acid motifs that are characteristic of fibroin and each consist of about 100 residues of amino acids, but are not limited thereto. The chemically modified artificial fibroin does not include those in which a partial structure not derived from an amino acid is introduced by chemical modification.

Herein, the term "domain sequence" is an amino acid sequence that generates a crystalline region (typically corresponding to an (A)ₙ motif of an amino acid sequence) and an amorphous region (typically corresponding to an REP of an amino acid sequence) specific to fibroin and is represented by Formula 1: [(A)ₙ motif-REP]ₘ or Formula 2: [(A)ₙ motif-REP]ₘ-(A)ₙ motif. Here, the (A)ₙ motif represents an amino acid sequence mainly consisting of alanine residues, and the number of amino acid residues is 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. In addition, the proportion of the number of alanine residues to the total number of amino acid residues in the (A)ₙ motif is 40% or more and may also be 60% or more, 70% or more, 80% or more, 83% or more, 85% or more, 86% or more, 90% or more, 95% or more, or 100% (which means that the (A)ₙ motif consists of alanine residues). At least seven of a plurality of (A)ₙ motifs in the domain sequence may consist of alanine residues. The "REP" is an amino acid sequence consisting of 2 to 200 amino acid residues. The "REP" may also be an amino acid sequence consisting of 10 to 200 amino acid residues. The symbol "m" represents an integer from 2 to 300 and may be an integer from 10 to 300. The plurality of (A)ₙ motifs may be identical or different amino acid sequences. The plurality of REPs may be the same amino acid sequence or different amino acid sequences.

Specific examples of the artificial fibroin include major dragline silk produced in major ampullate glands of spiders as described in WO 2021/187502.

Specific examples of the artificial fibroin include artificial fibroin derived from a protein (first artificial fibroin), artificial fibroin containing a domain sequence in which the content of glycine residues is reduced (second artificial fibroin), artificial fibroin containing a domain sequence in which the content of an (A)n motif is reduced (third artificial fibroin), artificial fibroin in which the content of glycine residues and the content of an (A)n motif are reduced (fourth artificial fibroin), artificial fibroin containing a domain sequence including a region locally having a high hydropathy index (fifth artificial fibroin), and artificial fibroin containing a domain sequence in which the content of glutamine residues is reduced (sixth artificial fibroin).

The artificial fibroin may have a tag sequence at one or both of the N-terminal and the C-terminal thereof. This makes it possible to isolate, immobilize, detect, and visualize the artificial fibroin.

Examples of the tag sequence include an affinity tag using specific affinity (binding properties or affinity) to another molecule. A specific example of the affinity tag includes a histidine tag (His-tag). The His-tag is a short peptide in which about 4 to 10 histidine residues are lined up and can be used for isolating artificial fibroin by chelating metal chromatography, since it has a property of specifically binding to metal ions such as nickel. Specific examples of the tag sequence include an amino acid sequence set forth in SEQ ID NO: 8 (an amino acid sequence including a His-tag sequence and a hinge sequence).

In addition, it is possible to employ a tag sequence such as glutathione-S-transferase (GST) that specifically binds to glutathione or a maltose binding protein (MBP) that specifically binds to maltose.

An "epitope tag" utilizing an antigen-antibody reaction is employable. Adding a peptide (epitope) exhibiting antigenicity as a tag sequence allows binding of an antibody to the epitope. Examples of the epitope tag include an HA (a peptide sequence of influenza virus hemagglutinin) tag, a myc tag, and a FLAG tag. The use of the epitope tag allows purification of the artificial fibroin to be easily performed with high specificity.

Furthermore, it is possible to use a tag sequence which is cleaved with a specific protease. By treating a protein adsorbed via the tag sequence with a protease, the artificial fibroin from which the tag sequence is cleaved can be recovered.

A specific example of the artificial fibroin includes artificial fibroin shown in Table 2.

**[Table 2]**

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7. 0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9. 7 | 0 | 7. 0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7. 0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |

The artificial fibroin may be artificial fibroin having at least two characteristics among the characteristics of the first artificial fibroin, the second artificial fibroin, the third artificial fibroin, the fourth artificial fibroin, the fifth artificial fibroin, and the sixth artificial fibroin.

### (Method for producing β-ketoester of protein)

A second embodiment of the present invention is a method for producing the β-ketoester of the protein according to the first embodiment. The method according to the present embodiment includes a step (hereinafter, referred to as "β-ketoesterification step") of mixing a protein containing an amino acid having a hydroxyl group and a compound represented by Formula (2a) or (2b) in a solvent and performing stirring while heating: 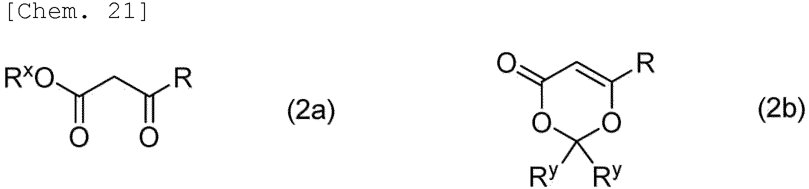 wherein R represents a C₁₋₆ alkyl group or an optionally substituted aryl group, and R^{x} and R^{y} each independently represent a C₁₋₆ alkyl group or an optionally substituted aryl group.

The definition and preferred embodiment of the protein containing an amino acid having a hydroxy group can refer to the first embodiment.

In Formulas (2a) and (2b), R is a C₁₋₆ alkyl group or an optionally substituted aryl group. Examples of the C₁₋₆ alkyl group include a methyl group, an ethyl group, a 1-propyl group, a 2-propyl group, a 1-butyl group, a 2-butyl group, a 1-pentyl group, a 2-pentyl group, a 3-pentyl group, a neopentyl group, a 1-hexyl group, a 2-hexyl group, and a 3-hexyl group. Examples of the aryl group include aromatic hydrocarbon ring groups such as a phenyl group and a naphthalene group, and heterocyclic groups such as a pyridyl group, a pyrimidinyl group, a pyrazinyl group, a furanyl group, a thiophenyl group, a pyrrole group, a quinolinyl group, and an isoquinyl group. The aryl group may be further substituted with one or more groups selected from a C₁₋₆ alkyl group, a C₁₋₆ alkoxy group, a halogen atom, a sulfo group, and a carboxy group.

In Formulas (2a) and (2b), R^{x} and R^{y} are each independently a C₁₋₆ alkyl group or an optionally substituted aryl group. Examples of the C₁₋₆ alkyl group include a methyl group, an ethyl group, a 1-propyl group, a 2-propyl group, a 1-butyl group, a 2-butyl group, a 1-pentyl group, a 2-pentyl group, a 3-pentyl group, a neopentyl group, a 1-hexyl group, a 2-hexyl group, and a 3-hexyl group. Examples of the aryl group include aromatic hydrocarbon ring groups such as a phenyl group and a naphthalene group, and heterocyclic groups such as a pyridyl group, a pyrimidinyl group, a pyrazinyl group, a furanyl group, a thiophenyl group, a pyrrole group, a quinolinyl group, and an isoquinyl group. The aryl group may be further substituted with one or more groups selected from a C₁₋₆ alkyl group, a C₁₋₆ alkoxy group, a halogen atom, a sulfo group, and a carboxy group. R¹ is preferably a secondary alkyl group or a tertiary alkyl group, and more preferably a tertiary alkyl group.

The use amount of the compound represented by Formula (2a) or (2b) may be 0.3 to 2.0 parts by mass, 0.3 to 1.8 parts by mass, 0.3 to 1.5 parts by mass, 0.3 to 1.3 parts by mass, 0.3 to 1.0 parts by mass, 0.4 to 2.0 parts by mass, 0.4 to 1.8 parts by mass, 0.4 to 1.5 parts by mass, 0.4 to 1.3 parts by mass, 0.4 to 1.0 parts by mass, 0.5 to 2.0 parts by mass, 0.5 to 1.8 parts by mass, 0.5 to 1.5 parts by mass, 0.5 to 1.3 parts by mass, or 0.5 to 1.0 parts by mass with respect to 1 part by mass of the protein. As the use amount of the compound represented by Formula (2a) or (2b) is larger, the number of β-ketoester skeletons to be introduced is larger, so that the solubility in an aprotic polar solvent and the coagulability can be excellent.

In the β-ketoesterification step, first, an aprotic polar solvent is added to the protein as a raw material, and the protein is dissolved by heating as necessary. The aprotic polar solvent may be any solvent as long as it does not inhibit or suppress the β-ketoesterification reaction, and examples thereof include DMSO, DMF, DMAc, and NMP. The use amount of the aprotic polar solvent may be 1.5 parts by mass to 999 parts by mass, and is preferably 2 parts by mass to 999 parts by mass, 4 parts by mass to 999 parts by mass, or 4 parts by mass to 99 parts by mass, with respect to 1 part by mass of the use amount of the protein. The heating temperature for dissolving the protein may be any temperature at which the protein is not thermally decomposed, and may be, for example, 20 to 180°C, 40 to 180°C, or 40 to 150°C.

Next, the compound represented by Formula (2a) or (2b) is added to the obtained protein solution, and the mixture is further stirred under heating conditions. The use amount of the compound represented by Formula (2a) or (2b) may be 0.01 parts by mass to 10 parts by mass, and is preferably 0.1 parts by mass to 10 parts by mass, 0.1 parts by mass to 7 parts by mass, or 0.1 parts by mass to 5 parts by mass with respect to 1 part by mass of the protein. The temperature of the β-ketoesterification reaction is usually 30 to 190°C, and may be 50 to 190°C or 50 to 160°C. The β-ketoesterification reaction may be stopped when disappearance of the protein as a raw material is confirmed, or may be stopped when a predetermined time elapses. The β-ketoester reaction is usually terminated in 1 to 2 hours.

The reaction can be stopped by adding acetone to the reaction solution to precipitate a product. Purification can be performed by utilizing methods commonly used in protein purification. For example, a method in which the precipitated product is centrifuged to distill off the solvent, or a method in which the precipitated product is collected by filtration, washed with a solvent, and dried under reduced pressure can be employed.

According to the method according to the present embodiment, a protein can be β-ketoesterified by a simple method. Since a protein has low solubility, a solvent that can be used in chemical modification is limited, and DMSO or HFIP is generally used. However, HFIP may react with the acylating agent described above. In addition, when DMSO is used as a solvent, under the acylation reaction conditions using an acylating agent (for example, acid halides and acid anhydrides) often used in the organic chemical field, the Swern oxidation type reaction proceeds to oxidize the hydroxyl group, and thus it is difficult to obtain a target product. However, in the method according to the present embodiment, since the reaction proceeds while the compound represented by Formula (2a) or (2b) is decomposed, a desired β-ketoester can be obtained by a simple method of mixing and heating in a solvent. In addition, by-products generated by the reaction can also be easily removed by distilling off the solvent or filtering.

A third embodiment of the present invention is a method for producing a chemically modified protein having a group represented by Formula (3), the method including a step of mixing the β-ketoester of the protein according to the first embodiment and a compound represented by Formula (4) in a solvent: 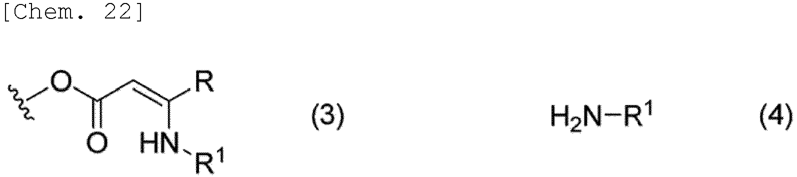 wherein R represents a C₁₋₆ alkyl group or an optionally substituted aryl group, and R¹ represents an organic group.

In the present embodiment, compounds represented by Formulas (3) and (4) are reacted with the β-ketoester of the protein according to the first embodiment to form an enamine, thereby introducing various functional chemical structures.

The compound represented by Formula (4) may be a primary amine capable of forming an enamine.

In Formula (4), R¹ is an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group. R¹ is preferably an organic group that does not inhibit or suppress enamine formation, and is preferably, for example, an organic group having no electron withdrawing group at the 1st to 2nd carbon atom from the nitrogen atom of the amino group.

The compound represented by Formula (4) preferably has a functional group. The functional group of the compound represented by Formula (4) is introduced into a protein, whereby the corresponding function can be imparted to the protein.

In the step of mixing the β-ketoester of the protein and the compound represented by Formula (4) in a solvent, the use amount of the compound represented by Formula (4) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which enamine formation proceeds, and may be, for example, 0 to 95°C, 5 to 90°C, 10 to 90°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

Specific examples of R¹ are shown in Table 3.

**[Table 3]**

| Examples of R¹ | Specific examples | Function obtained by introduction of functional group |
|---|---|---|
| Fluorescent group | 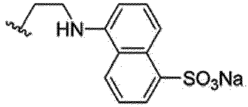 | Fluorescent labelling |
| 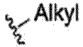 | 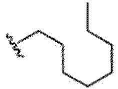 | Hydrophobicity, solubility in solvent, thermal stability (for example, increase or decrease in glass transition temperature Tg, expression of Tm) |
| 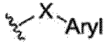 | 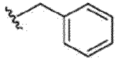 | Hydrophobicity, solubility in solvent |
| 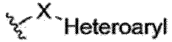 | 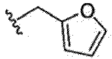 | Solubility in solvent |

In Table 3, X represents, for example, a C₁₋₂₀ alkylene group, a C₆₋₂₀ arylene group, a C₆₋₂₀ arylene group-C₁₋₂₀ alkylene group, a C₆₋₂₀ arylene group-C₁₋₂₀ alkylene group-C₆₋₂₀ arylene group, a C₁₋₂₀ alkylene group-C₆₋₂₀ arylene group-C₁₋₂₀ alkylene group, a polyethylene glycol group, a polypropylene glycol group, a polyethylene/propylene glycol group, and a tetramethylene glycol group.

The C₁₋₂₀ alkylene group is an alkylene group having 1 to 20 carbon atoms, and examples thereof include a methylene group, an ethylene group, a propylene group, a butylene group, a pentylene group, a hexylene group, a heptylene group, an octylene group, a nonylene group, a decylene group, an undecylene group, and a dodecylene group.

The C₆₋₂₀ arylene group is an arylene group having 6 to 20 carbon atoms, and examples thereof include a phenylene group, a naphthylene group, a terphenylene group, a pyrenylene group, and a biphenylene group.

The C₆₋₂₀ arylene group-C₁₋₂₀ alkylene group is a divalent group in which an alkylene having 1 to 20 carbon atoms and an arylene group having 6 to 20 carbon atoms are covalently bonded. The C₆₋₂₀ arylene group-C₁₋₂₀ alkylene group-C₆₋₂₀ arylene group and the C₁₋₂₀ alkylene group-C₆₋₂₀ arylene group-C₁₋₂₀ alkylene group are divalent groups in which an alkylene having 1 to 20 carbon atoms and an arylene group having 6 to 20 carbon atoms are covalently bonded. Specific examples thereof include a methylene diphenylene group and a phenylenebis(methylene) group.

A fourth embodiment of the present invention is a method for producing a chemically modified protein having a group represented by Formula (5), the method including a step of mixing the β-ketoester of the protein according to the first embodiment and a compound represented by Formula (6) : 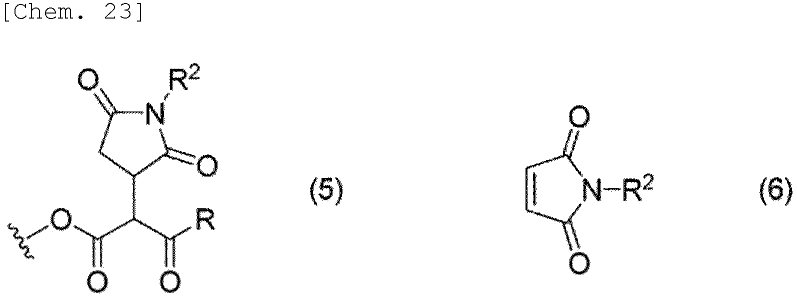 wherein R represents a C₁₋₆ alkyl group or an optionally substituted aryl group, and R² represents an organic group.

In the present embodiment, a compound represented by Formula (6) is reacted with the β-ketoester of the protein according to the first embodiment to thereby introduce various functional chemical structures through a Michael addition reaction.

The compound represented by Formula (6) may be an N-substituted maleimide. In Formulas (5) and (6), R² is an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (6) preferably has a functional group. The functional group of the compound represented by Formula (6) is introduced into a protein, whereby the corresponding function can be imparted to the protein.

In the step of mixing the β-ketoester of the protein and the compound represented by Formula (6) in a solvent, the use amount of the compound represented by Formula (6) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

A fifth embodiment of the present invention is a method for producing a chemically modified protein having a group represented by Formula (7), the method including a step of mixing the β-ketoester of the protein according to the first embodiment and a compound represented by Formula (8) : 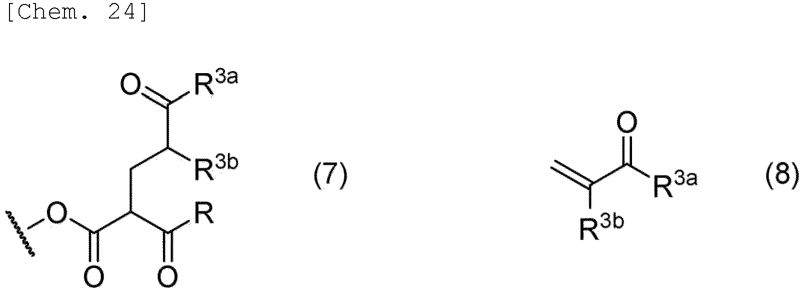 wherein R represents a C₁₋₆ alkyl group or an optionally substituted aryl group, and R^{3a} and R^{3b} each independently represent an organic group.

In the present embodiment, a compound represented by Formula (8) is reacted with the β-ketoester of the protein according to the first embodiment to thereby introduce various functional chemical structures through a Michael addition reaction.

The compound represented by Formula (8) may be an α, β-unsaturated carbonyl compound. In Formulas (7) and (8), R^{3a} is an organic group, preferably an organic group bonded via an oxygen atom, a nitrogen atom, a sulfur atom, or a carbon atom, or a hydrogen atom. Examples of the organic group include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (8) preferably has a functional group. The functional group of the compound represented by Formula (8) is introduced into a protein, whereby the corresponding function can be imparted to the protein.

In the step of mixing the β-ketoester of the protein and the compound represented by Formula (8) in a solvent, the use amount of the compound represented by Formula (8) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

A sixth embodiment of the present invention is a method for producing a chemically modified protein having a group represented by Formula (9), the method including a step of mixing the β-ketoester of the protein according to the first embodiment and a compound represented by Formula (10) in a solvent: 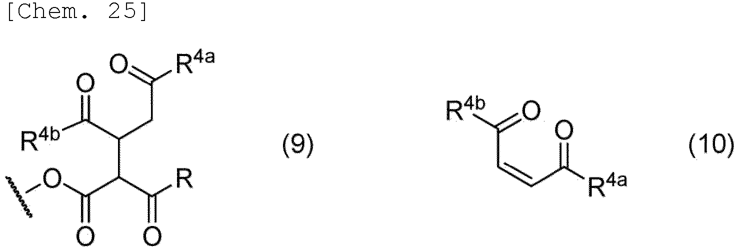 wherein R represents a C₁₋₆ alkyl group or an optionally substituted aryl group, and R^{4a} and R^{4b} each independently represent an organic group.

In the present embodiment, a compound represented by Formula (10) is reacted with the β-ketoester of the protein according to the first embodiment to thereby introduce various functional chemical structures through a Michael addition reaction.

In Formulas (9) and (10), R^{4a} and R^{4b} are each independently an organic group, and preferably an organic group bonded via an oxygen atom, a nitrogen atom, a sulfur atom, or a carbon atom, or a hydrogen atom. Examples of the organic group include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (10) preferably has a functional group. The functional group of the compound represented by Formula (10) is introduced into a protein, whereby the corresponding function can be imparted to the protein.

In the step of mixing the β-ketoester of the protein and the compound represented by Formula (10) in a solvent, the use amount of the compound represented by Formula (10) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

A seventh embodiment of the present invention is a method for producing a chemically modified protein having a group represented by Formula (11), the method including a step of mixing a β-ketoester of the protein according to the first embodiment and a compound represented by Formula (12) in a solvent: 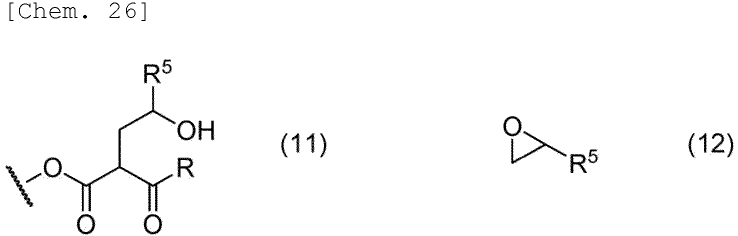 wherein R represents a C₁₋₆ alkyl group or an optionally substituted aryl group, and R⁵ represents an organic group.

In the present embodiment, a compound represented by Formula (12) is reacted with the β-ketoester of the protein according to the first embodiment to thereby introduce various functional chemical structures through an alkylation reaction.

The compound represented by Formula (12) may be an epoxide. In Formula (12), R⁵ is an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (12) preferably has a functional group. The functional group of the compound represented by Formula (12) is introduced into a protein, whereby the corresponding function can be imparted to the protein.

In the step of mixing the β-ketoester of the protein and the compound represented by Formula (12) in a solvent, the use amount of the compound represented by Formula (12) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

An eighth embodiment of the present invention is a method for producing a chemically modified protein having a group represented by Formula (13), the method including a step of mixing the β-ketoester of the protein according to the first embodiment and a compound represented by Formula (14) in a solvent: 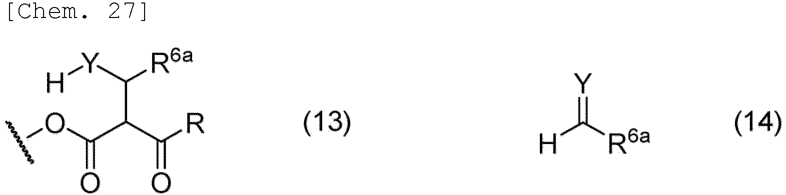 wherein R represents a C₁₋₆ alkyl group or an optionally substituted aryl group, R^{6a} represents an organic group, Y represents an oxygen atom or NR^{6b}, and R^{6b} represents an organic group.

In the present embodiment, a compound represented by Formula (6) is reacted with the β-ketoester of the protein according to the first embodiment to thereby introduce various functional chemical structures through a nucleophilic addition reaction.

When Y is an oxygen atom, the compound represented by Formula (14) is an aldehyde, and when Y is NR^{6b}, the compound represented by Formula (14) is an imine. In Formulas (13) and (14), R^{6a} and R^{6b} are each independently an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (14) preferably has a functional group. The functional group of the compound represented by Formula (14) is introduced into a protein, whereby the corresponding function can be imparted to the protein.

In the step of mixing the β-ketoester of the protein and the compound represented by Formula (14) in a solvent, the use amount of the compound represented by Formula (14) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Bronsted acid represented by acetic acid or the like, or a Bronsted base having basicity but having no nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

A ninth embodiment of the present invention is a method for producing a chemically modified protein having a group represented by Formula (15), the method including a step of mixing the β-ketoester of the protein according to the first embodiment and a compound represented by Formula (16) in a solvent: 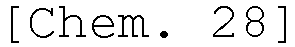 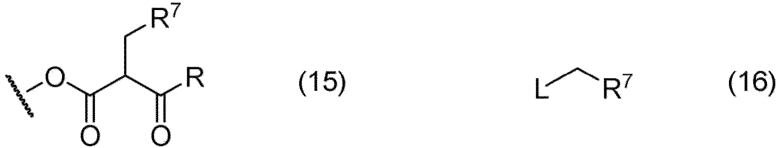 wherein R represents a C₁₋₆ alkyl group or an optionally substituted aryl group, R⁷ represents an organic group, and L represents a leaving group.

In the present embodiment, a compound represented by Formula (16) is reacted with the β-ketoester of the protein according to the first embodiment to thereby introduce various functional chemical structures through an alkylation reaction.

In Formulas (15) and (16), R⁷ is an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

In Formulae (15) and (16), L represents a leaving group, and examples thereof include alkyl groups or aryl groups bonded via a halogen atom (for example, a fluorine atom, a chlorine atom, a bromine atom, or an iodine atom), an oxycarbonyl group or an oxysulfonyl group. The alkyl group and the aryl group are preferably further substituted with an electron withdrawing group (for example, a halogen atom, a nitro group, or a halogenated alkyl group).

The compound represented by Formula (16) preferably has a functional group. The functional group of the compound represented by Formula (16) is introduced into a protein, whereby the corresponding function can be imparted to the protein.

In the step of mixing the β-ketoester of the protein and the compound represented by Formula (16) in a solvent, the use amount of the compound represented by Formula (16) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 20 to 100°C, 25 to 95°C, 30 to 93°C, or 35 to 90°C.

In the above step, in order to shorten the reaction time, a catalytic amount of a Bronsted base having basicity but not having nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

A tenth embodiment of the present invention is a method for producing a chemically modified protein having a group represented by Formula (17), the method including a step of mixing the β-ketoester of the protein according to the first embodiment and a compound represented by Formula (18) in a solvent: 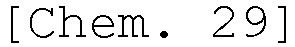 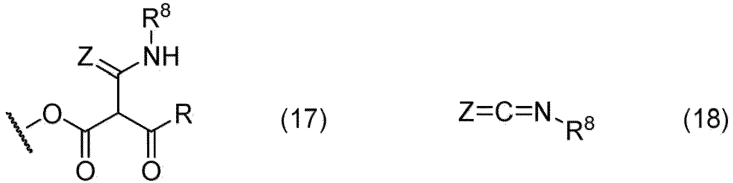 wherein R represents a C₁₋₆ alkyl group or an optionally substituted aryl group, R⁸ represents an organic group, and Z represents an oxygen atom or a sulfur atom.

In the present embodiment, a compound represented by Formula (18) is reacted with the β-ketoester of the protein according to the first embodiment to thereby introduce various functional chemical structures.

The compound represented by Formula (18) may be isocyanate or thioisocyanate. In Formulas (17) and (18), R⁸ is an organic group, and examples thereof include a chain or branched alkyl group, a cycloalkyl group, an aryl group, and a heterocyclic group. These groups may be further substituted with one or more groups selected from a halogen atom, a carboxy group, a hydroxyl group, an amino group, a chain or branched alkyl group, a cycloalkyl group, an aryl group, and a heterocyclic group.

The compound represented by Formula (18) preferably has a functional group. The functional group of the compound represented by Formula (18) is introduced into a protein, whereby the corresponding function can be imparted to the protein.

In the step of mixing the β-ketoester of the protein and the compound represented by Formula (18) in a solvent, the use amount of the compound represented by Formula (18) may be 0.01 to 500 mol, 0.03 to 450 mol, 0.05 to 400 mol, or 0.1 to 350 mol, with respect to 1 mol of the use amount of the β-ketoester of the protein.

The above step is preferably performed in an aprotic polar solvent. Examples of the aprotic polar solvent include dimethyl sulfoxide (DMSO), N,N-dimethylformamide (DMF), N,N-dimethylacetamide (DMAc), and N-methylpyrrolidone (NMP).

The step may be performed at a temperature at which the addition reaction proceeds, and may be, for example, 0 to 100°C, 5 to 95°C, 10 to 93°C, or 15 to 90°C.

In the above step, in order to shorten the reaction time, a catalytic amount of a Lewis acid represented by dibutyltin dilaurate or the like, or a Bronsted base having basicity but not having nucleophilicity, represented by triethylamine or the like, may be added.

Depending on the type of functional group, functions such as a fluorescent labeling function, hydrophobicity, solubility in a solvent, and thermal stability (for example, increase or decrease in glass transition temperature Tg) can be imparted to the protein. The degree of such functions can also be adjusted by adjusting the number of functional groups to be introduced.

### Examples

### 1. Production of artificial protein

### (1) Preparation of expression vector

An artificial protein having an amino acid sequence set forth in SEQ ID NO: 2 (PRT966), an artificial protein having an amino acid sequence set forth in SEQ ID NO: 5 (PRT1000), an artificial protein having an amino acid sequence set forth in SEQ ID NO: 6 (PRT1001), and an artificial protein having an amino acid sequence set forth in SEQ ID NO: 7 (PRT587) were designed. The value of the average hydropathy index of the artificial protein having an amino acid sequence set forth in SEQ ID NO: 2 (PRT966) is 0.44, the value of the average hydropathy index of the artificial protein having an amino acid sequence set forth in SEQ ID NO: 5 (PRT1000) is 0.45, the value of the average hydropathy index of the artificial protein having an amino acid sequence set forth in SEQ ID NO: 6 (PRT1001) is 0.46, and the value of the average hydropathy index of the artificial protein having an amino acid sequence set forth in SEQ ID NO: 7 (PRT587) is -0.8. The proportions of total of serine residues, threonine residues, and tyrosine residues in PRT587 and PRT966 are all 16.5%. Any of the artificial proteins is an artificial fibroin designed based on a naturally derived fibroin.

The amino acid sequence set forth in SEQ ID NO: 5 (PRT1000) is obtained by inserting one cysteine residue into an REP located at position 1 on each of the N-terminal side and the C-terminal side of a domain sequence in the amino acid sequence set forth in SEQ ID NO: 2 (PRT966). The total number of cysteine residues in the artificial protein having the amino acid sequence set forth in SEQ ID NO: 5 is 2. The amino acid sequence set forth in SEQ ID NO: 6 (PRT1001) is obtained by inserting one cysteine residue into an REP located at positions 1 to 2 on each of the N-terminal side and the C-terminal side of a domain sequence in the amino acid sequence set forth in SEQ ID NO: 2 (PRT966). The total number of cysteine residues in the artificial protein having the amino acid sequence set forth in SEQ ID NO: 6 is 4. The amino acid sequence set forth in SEQ ID NO: 7 (PRT587) is obtained by acquiring a base sequence and an amino acid sequence of fibroin (GenBank Accession No: P46804.1, GI: 1174415) derived from *Nephila clavipes* from the web database GenBank, then performing substitution, insertion, and deletion of amino acid residues for improving productivity, and further adding an amino acid sequence (tag sequence and hinge sequence) set forth in SEQ ID NO: 8 to the N-terminal.

Each of nucleic acids encoding artificial proteins having the amino acid sequences set forth in SEQ ID NO: 2, SEQ ID NO: 5, SEQ ID NO: 6, and SEQ ID NO: 7 designed as described above was synthesized. In each of the nucleic acids, an NdeI site was added to a 5' terminal and an EcoRI site was added downstream of a stop codon. The nucleic acid was cloned into a cloning vector (pUC118). Thereafter, the nucleic acid was enzymatically cleaved by treatment with NdeI and EcoRI, and then, recombined into the protein expression vector pET-22b(+), thereby obtaining an expression vector.

### (2) Expression of protein

*Escherichia coli* BLR (DE3) was transformed with the obtained expression vector. The transformed *Escherichia coli* was cultured in 2 mL of an LB culture medium containing ampicillin for 15 hours. The culture solution was added to a 100 mL seed culture medium containing ampicillin (Table 4) so that OD₆₀₀ was 0.005. The culture solution was maintained at a temperature of 30°C, and placed in a culture flask (for about 15 hours) until OD₆₀₀ reached 5, thereby obtaining a seed culture solution.

**[Table 4]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter to which a 500 mL production medium (Table 5) was added so that OD₆₀₀ was 0.05. Culture was performed while maintaining the culture solution at a temperature of 37°C and controlling the pH to be constant at 6.9. Furthermore, the culture solution was maintained to have a 20% dissolved oxygen concentration of the saturated dissolved oxygen concentration.

**[Table 5]**

| Production medium | |
|---|---|
| Reagent | Concentration (g/L) |
| Glucose | 12.0 |
| KH₂PO₄ | 9.0 |
| MgSO₄.7H₂O | 2.4 |
| Yeast Extract | 15 |
| FeSO₄.7H₂O | 0.04 |
| MnSO₄.5H₂O | 0.04 |
| CaCl₂.2H₂O | 0.04 |
| ADEKA NOL (ADEKA, LG-295S) | 0.1 (mL/L) |

Immediately after glucose in the production medium was completely consumed, a feed solution (455 g/1 L of glucose, 120 g/1 L of Yeast Extract) was added to the production medium at a speed of 1 mL/min. Culture was performed while maintaining the culture solution at a temperature of 37°C and controlling the pH to be constant at 6.9. The culture was performed for 20 hours while a dissolved oxygen concentration in the culture solution was maintained at 20% of the saturated dissolved oxygen concentration. Thereafter, 1 M isopropyl-β-thiogalactopyranoside (IPTG) was added to the culture solution so that the final concentration thereof was 1 mM, thus inducing the expression of the artificial protein. When 20 hours had elapsed after the addition of IPTG, the culture solution was centrifuged, and bacterial cells were collected. SDS-PAGE was performed using bacterial cells prepared from the culture solutions before and after the addition of IPTG, and expression of the target artificial protein was confirmed by appearance of a band of the size of the target artificial protein depending on the addition of IPTG.

### (3) Purification of protein

Bacterial cells that were collected 2 hours after the addition of IPTG were washed with a 20 mM Tris-HCl buffer (pH 7.4). The washed bacterial cells were suspended in a 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the precipitate reached a high level of purity. The washed precipitate was suspended in an 8 M guanidine buffer (8 M guanidine hydrochloride, 10 mM sodium dihydrogen phosphate, 20 mM NaCl, 1 mM Tris-HCl, pH 7.0) so that the precipitate reached a concentration of 100 mg/mL, and then, the precipitate was dissolved by being stirred with a stirrer at 60°C for 30 minutes. After the precipitate was dissolved, the resulting solution was dialyzed with water using a dialysis tube (cellulose tube 36/32 manufactured by Sanko Junyaku Co., Ltd.). A white aggregated protein obtained after dialysis was collected by centrifugation, and water was removed in a freeze dryer to recover freeze-dried powder, thereby obtaining three types of artificial proteins (PRT1000, PRT1001, PRT966, and PRT587).

### 2. Acetoacetylation of artificial protein

### (1) Artificial protein PRT966 (number average molecular weight: 100,000)

The artificial protein PRT966 was vacuum-dried to remove moisture. The dried artificial protein (1.0 g) was weighed in a stainless steel vial, and DMSO (19.0 g) was added thereto. Then, the mixture was stirred at 100°C for 30 minutes to dissolve the artificial protein, thereby obtaining a reaction solution. The resulting reaction solution was heated to 110°C, tert-butyl acetoacetate (1.0 g) was added to the reaction solution with stirring, and the mixture was further reacted for 1 hour. Acetone (60 g) was added to the reaction solution to precipitate a product, the product was centrifuged, and then the solvent was distilled off. This step was repeated three times in total to perform washing, and the resultant was dried under reduced pressure, thereby obtaining acetoacetylated PRT966. DMSO, unreacted tert-butyl acetoacetate, and tert-butyl alcohol were removed by the washing operation.

### (2) Artificial protein PRT587 (number average molecular weight: 100,000)

The artificial protein PRT587 of SEQ ID NO: 7 was vacuum-dried to remove moisture. The dried artificial protein (1.0 g) was weighed in a stainless steel vial, and DMSO (19.0 g) was added thereto. Then, the mixture was stirred at 100°C for 30 minutes to dissolve the artificial protein, thereby obtaining a reaction solution. The resulting reaction solution was heated to 110°C, tert-butyl acetoacetate (1.0 g) was added to the reaction solution with stirring, and the mixture was further reacted for 1 hour. Acetone (60 g) was added to the reaction solution to precipitate a product, the product was centrifuged, and then the solvent was distilled off. This step was repeated three times in total to perform washing, and the resultant was dried under reduced pressure, thereby obtaining acetoacetylated PRT587. DMSO, unreacted tert-butyl acetoacetate, and tert-butyl alcohol were removed by the washing operation.

### (3) Silk

Silk (1.0 g, trade name: Silk Powder IM, manufactured by KB SEIREN, Ltd.) was weighed in a stainless steel vial, and a DMSO (19.0 g) solution of lithium chloride (0.76 g, manufactured by FUJIFILM Wako Pure Chemical Corporation) was added thereto. Then, the mixture was stirred at 100°C for 30 minutes to dissolve the silk powder IM, thereby obtaining a reaction solution. The resulting reaction solution was heated to 110°C, tert-butyl acetoacetate (1.0 g) was added to the reaction solution with stirring, and the mixture was further reacted for 1 hour.

Acetone (60 g) was added to the reaction solution to precipitate a product, the product was centrifuged, and then the solvent was distilled off. This step was repeated three times in total to perform washing, and the resultant was dried under reduced pressure, thereby obtaining acetoacetylated silk powder. DMSO, unreacted tert-butyl acetoacetate, and tert-butyl alcohol were removed by the washing operation.

### (4) Casein

Casein (1.0 g, manufactured by FUJIFILM Wako Pure Chemical Corporation) was weighed in a stainless steel vial, and DMSO (19.0 g) was added thereto. Then, the mixture was stirred at 100°C for 30 minutes to dissolve the casein, thereby obtaining a reaction solution. The resulting reaction solution was heated to 110°C, tert-butyl acetoacetate (1.0 g) was added to the reaction solution with stirring, and the mixture was further reacted for 1 hour.

Acetone (60 g) was added to the reaction solution to precipitate a product, the product was centrifuged, and then the solvent was distilled off. This step was repeated three times in total to perform washing, and the resultant was dried under reduced pressure, thereby obtaining acetoacetylated silk powder. DMSO, unreacted tert-butyl acetoacetate, and tert-butyl alcohol were removed by the washing operation.

### (5) Collagen

Collagen (1.0 g, trade name: Nippi collagen 100, manufactured by Nippi. Inc.) was weighed in a stainless steel vial, and DMSO (19.0 g) was added thereto. Then, the mixture was stirred at 100°C for 30 minutes to dissolve the collagen, thereby obtaining a reaction solution. The resulting reaction solution was heated to 110°C, tert-butyl acetoacetate (1.0 g) was added to the reaction solution with stirring, and the mixture was further reacted for 1 hour.

Acetone (60 g) was added to the reaction solution to precipitate a product, the product was centrifuged, and then the solvent was distilled off. This step was repeated three times in total to perform washing, and the resultant was dried under reduced pressure, thereby obtaining acetoacetylated silk powder. DMSO, unreacted tert-butyl acetoacetate, and tert-butyl alcohol were removed by the washing operation.

### 3. Scale-up of acetoacetylation reaction of artificial protein PRT966

In a 3,000 mL three-necked flask, the artificial protein PRT966 (100 g) having an amino acid sequence of SEQ ID NO: 2 and DMSO (1,900 g) were weighed, and a temperature sensor, a reflux tube, and a silicon stopper were set to the flask. The flask was heated to 100°C using an oil bath containing silicon oil. The artificial protein was dissolved by stirring at that temperature for 30 minutes to obtain a reaction solution. The resulting reaction solution was heated to 110°C, tert-butyl acetoacetate (100 g) was then added to the reaction solution little by little, and the mixture was reacted for 1 hour.

Acetone (6,000 g) was added to the reaction solution to precipitate a product. The precipitate was pulverized using a three-one motor and a homogenizer, the pulverized product was centrifuged, and then the solvent was distilled off. This step was repeated three times in total to perform washing, and the resultant was dried under reduced pressure, thereby obtaining an acetoacetylated artificial protein. DMSO, unreacted tert-butyl acetoacetate, and tert-butyl alcohol were removed by washing operation.

### <Analysis of product>

Acetoacetylation of the protein (artificial protein, silk, casein, collagen) was confirmed by nuclear magnetic resonance spectrum (NMR), infrared spectrum (IR), gel permeation chromatography (GPC), and electrophoresis (SDS-PAGE) .

### (1)¹H-NMR

In a glass container, 50 mg of an acetoacetylated protein (artificial protein, silk, casein, collagen) or corresponding unreacted protein (control) was placed, DMSO-d6 (1,090 µL, manufactured by Eurisotop) was added thereto, and the mixture was heated at 90°C for dissolution. The obtained solution was transferred to an NMR measurement tube (manufactured by FUJIFILM Wako Pure Chemical Corporation), and subjected to NMR measurement. It is noted that, in the measurement of acetoacetylated silk and silk, lithium chloride (48 mg, manufactured by FUJIFILM Wako Pure Chemical Corporation) was further added to the glass container.

### [Measurement conditions]

Apparatus name: JNM-ECZ400R (manufactured by JEOL Ltd.)
Frequency: 400 MHz
Solvent: DMSO-d₆

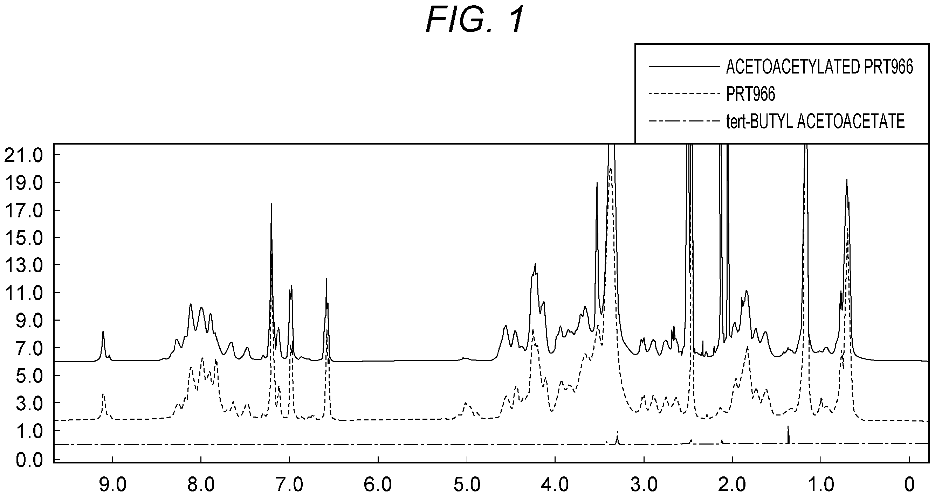

As shown in Fig. 1, in the ¹H-NMR spectrum of the acetoacetylated PRT966, peaks derived from the acetoacetyl group (3.5 ppm, 2.13 ppm) were observed. In addition, as compared with the unreacted PRT966, the peak (around 4.9 to 5.1 ppm) derived from the hydroxy group used in the acylation reaction was reduced.

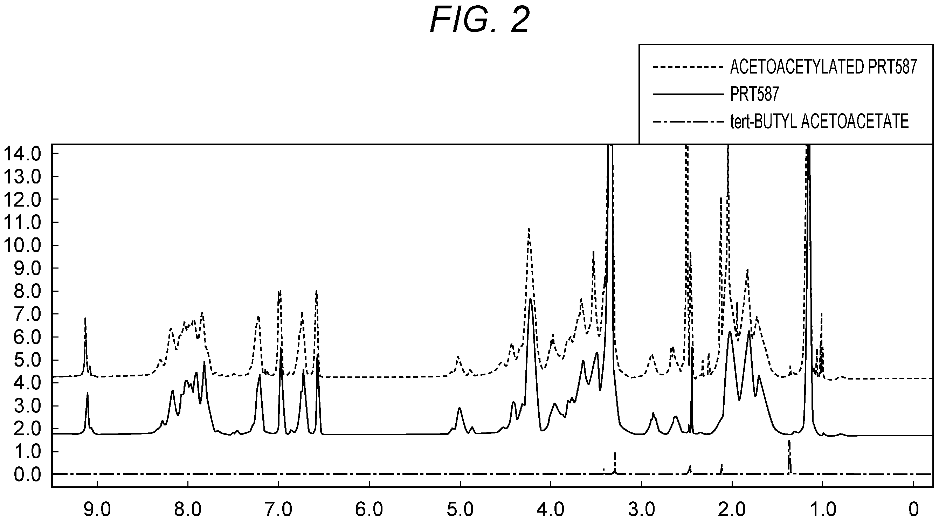

As shown in Fig. 2, in the ¹H-NMR spectrum of the acetoacetylated PRT587, a peak (3.5 ppm) derived from the methylene group of the acetoacetyl group was observed.

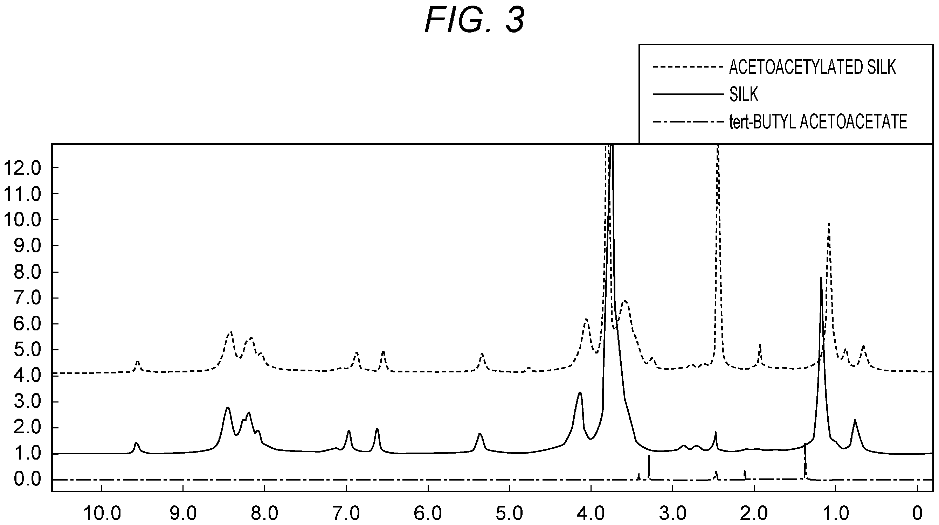

As shown in Fig. 3, in the ¹H-NMR spectrum of the acetoacetylated silk, several new peaks were observed, but the peaks were broad, and thus it was not possible to assign peaks to hydrogen atoms from which these peaks are derived.

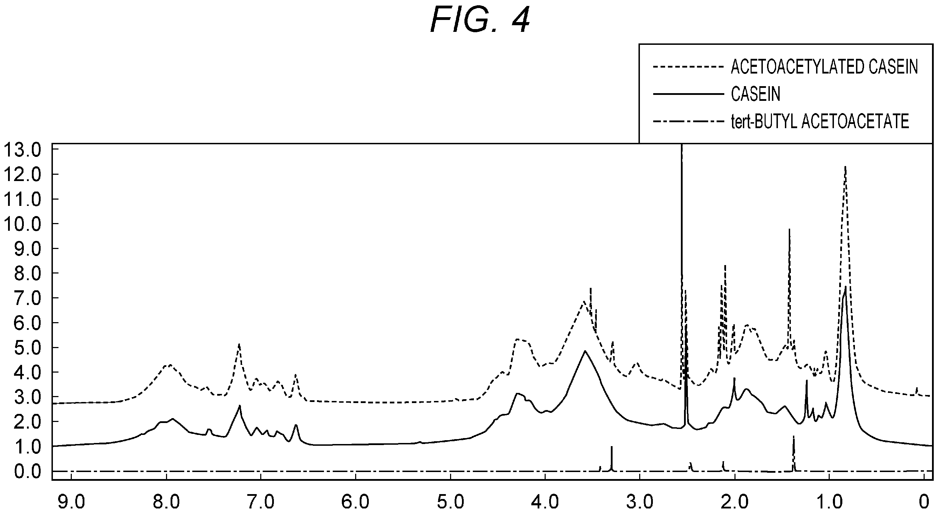

As shown in Fig. 4, in the ¹H-NMR spectrum of the acetoacetylated casein, peaks derived from the acetoacetyl group were observed at 3.6 ppm and 2.2 ppm.

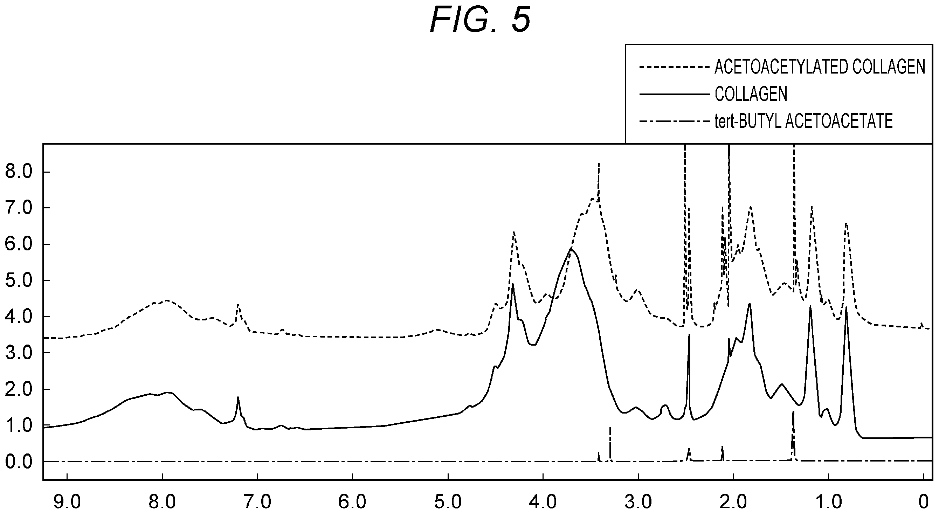

As shown in Fig. 5, in the ¹H-NMR spectrum of the acetoacetylated collagen, a peak derived from the acetoacetyl group was observed at 2.0 ppm.

### (2) IR

The infrared spectrum of the acetoacetylated protein (artificial protein, silk, casein, collagen) was measured using a Fourier transform infrared spectrophotometer.

### [Measurement conditions]

Apparatus name: Nicolet iS50 FT-IR (manufactured by Thermo Fisher Scientific Inc.)
Measurement method: ATR method (total reflection method)

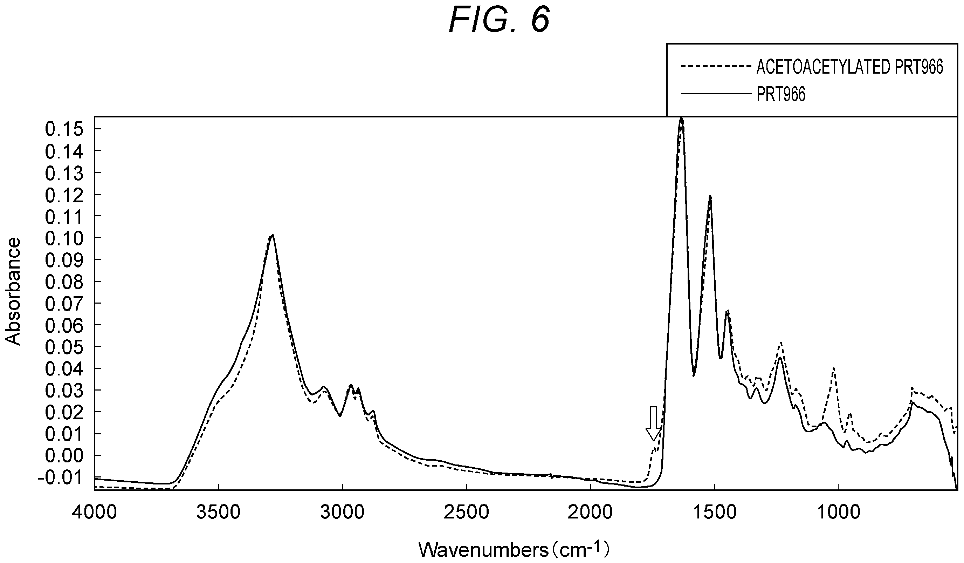

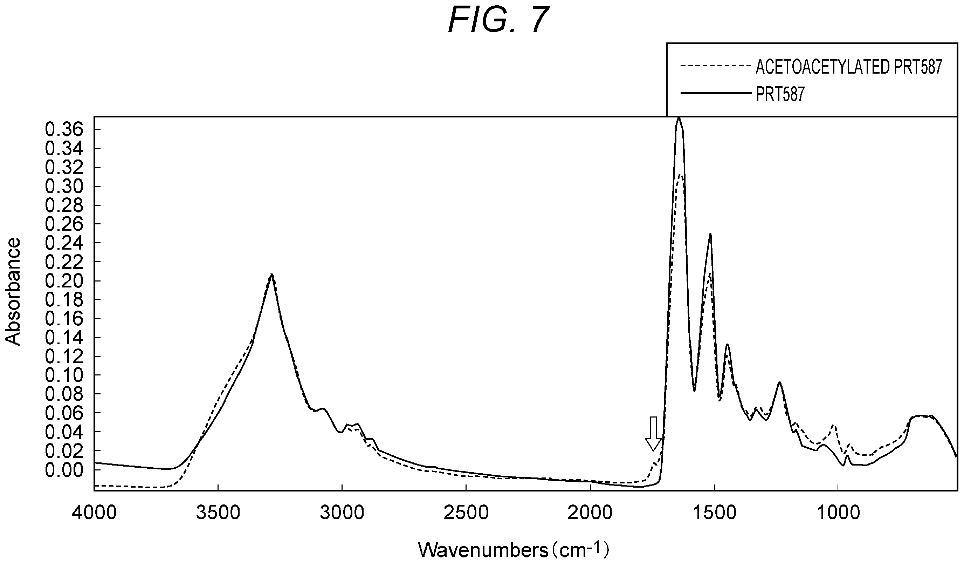

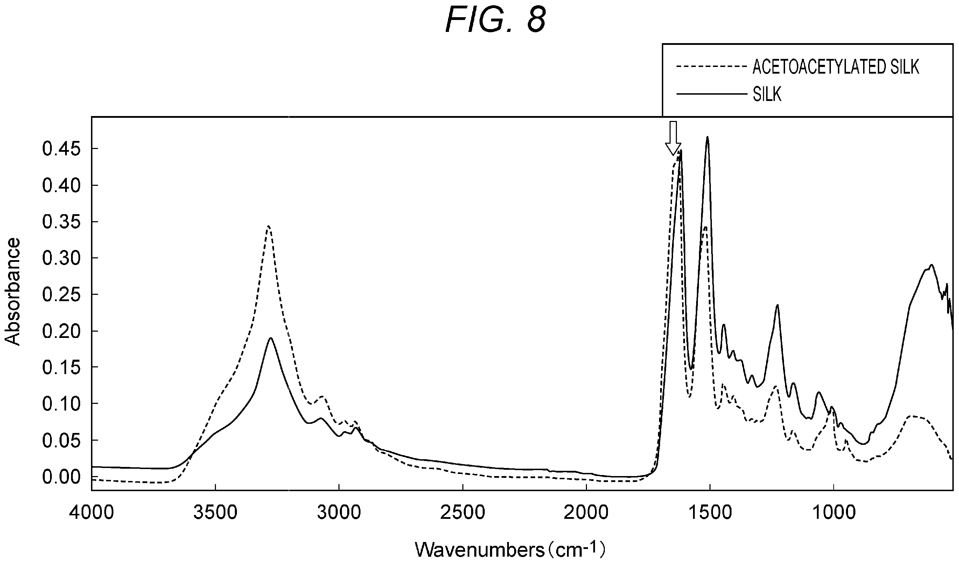

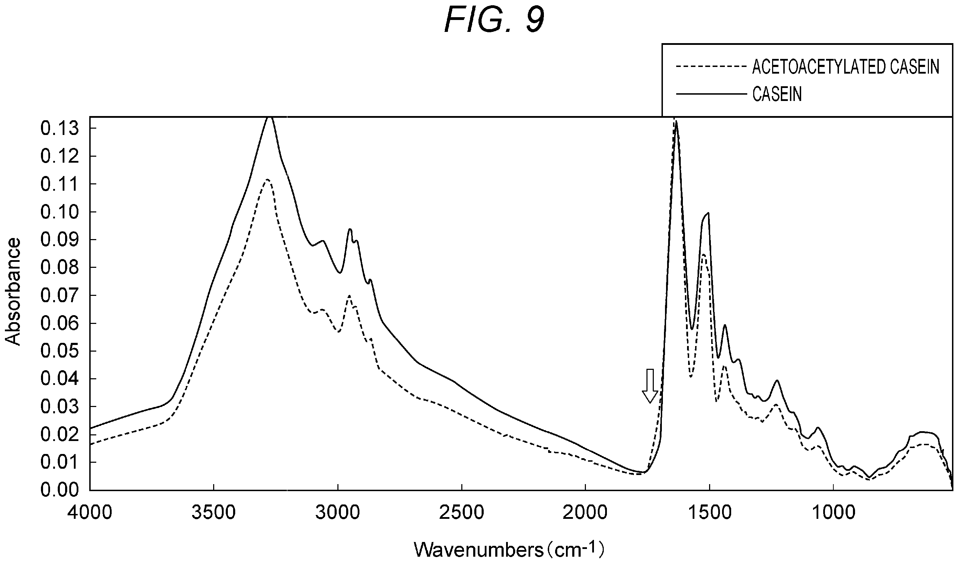

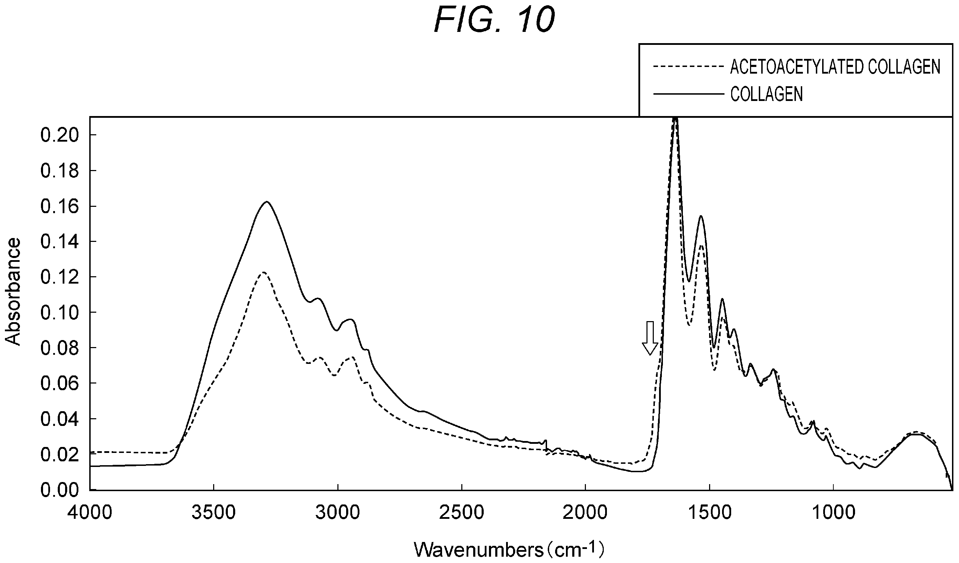

The IR spectra of the acetoacetylated proteins are shown in Fig. 6 to 10. As indicated by the arrow in each figure, in the spectrum of the acetoacetylated artificial protein, casein, or collagen, an ester side carbonyl peak of the acetoacetyl group was observed at 1,720 cm⁻¹. In the spectrum of the acetoacetylated silk, a similar peak was observed at 1,650 cm⁻¹. From the detection of these peaks, it was presumed that acetoacetylation of each protein proceeded.

### (3)GPC

The acetoacetylated protein (1.6 mg) was dissolved in HFIP (1.0 mL), and the obtained solution was measured by gel permeation chromatography. Two downsizing columns for rapid analysis (trade name: HFIP-606M (manufactured by Shodex), inner diameter: 6.0 mm × length 150 mm) were connected, and used as the column.

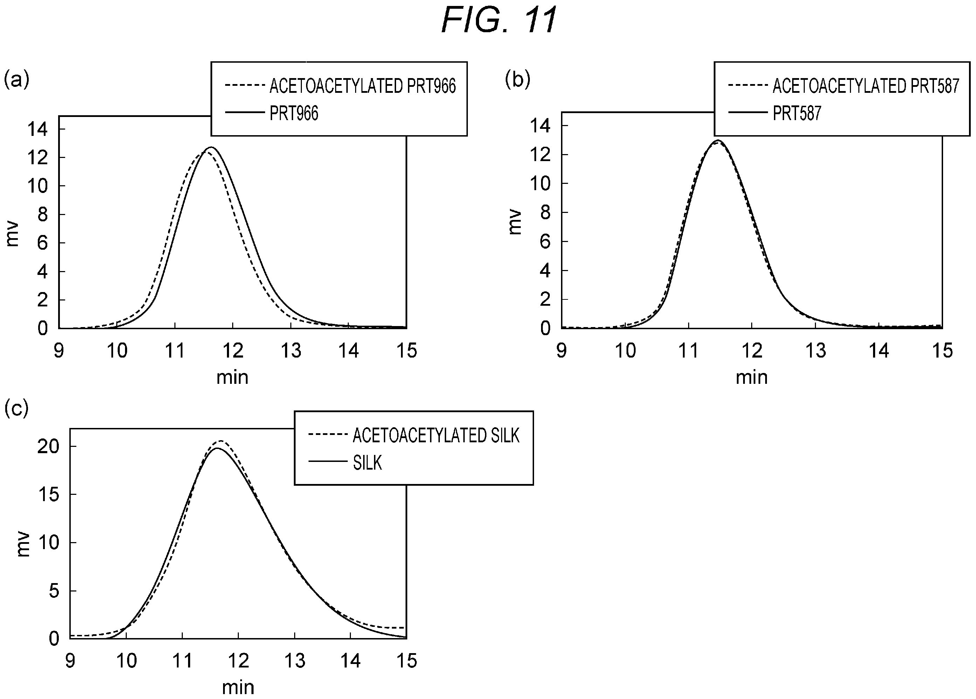

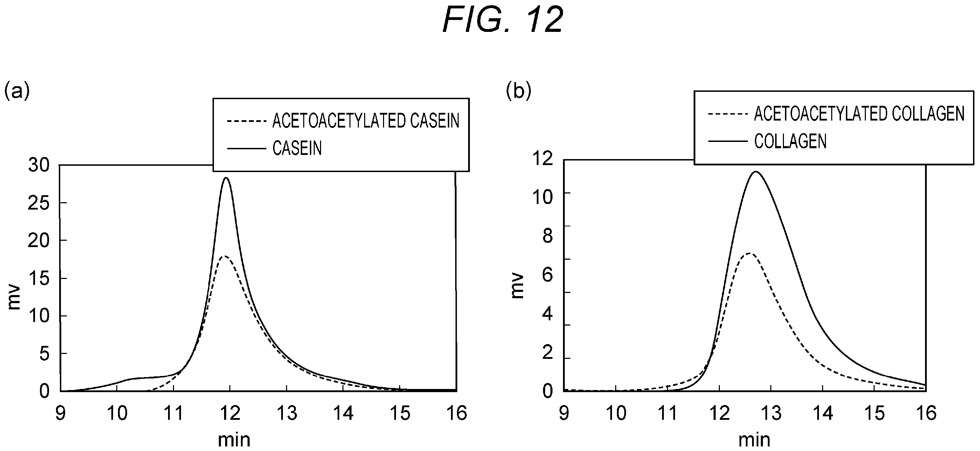

The chromatograms of the acetoacetylated proteins are shown in Figs. 11 and 12. Figs. 11(a) to (c) are chromatograms of PRT966 and acetoacetylated PRT966, PRT587 and acetoacetylated PRT587, silk and acetoacetylated silk in this order. Figs. 12(a) and (b) are chromatograms of casein and acetoacetylated casein, and collagen and acetoacetylated collagen in this order. The vertical axis of the chromatogram represents the detection concentration of the differential refractive index detector (RI), and the horizontal axis represents the elution time. A decrease in molecular size of the protein could not be confirmed from each chromatogram, and thus it was confirmed that the degradation reaction did not occur.

### (4)SDS-PAGE

In a glass container, 2 mg of an acetoacetylated protein (artificial protein, silk, casein, collagen) or corresponding unreacted protein (control) was placed, 2 M lithium chloride-containing DMSO (200 µL) was added thereto, and the mixture was heated at 80°C for 1 hour and further heated at 95°C for 10 minutes for dissolution. Thereafter, the obtained solution was diluted 15 times with a 10 M aqueous urea solution, further diluted 2 times with a sample buffer solution (with 3-mercapto-1,2-propanediol (× 2) (trade name), manufactured by FUJIFILM Wako Pure Chemical Corporation), and treated at 95°C for 5 minutes. The treated sample (10 µL) was added to an SDS-PAGE gel and subjected to electrophoresis with an electrophoresis apparatus. After the electrophoresis, the SDS-PAGE gel was stained with a stain, and a fluorescence image was obtained by an analyzer.

### [Analysis conditions]

Apparatus name: SDS-PAGE electrophoresis apparatus (manufactured by Bio-Rad Laboratories, Inc.)
Gel: SDS-PAGE gel (manufactured by Bio-Rad Laboratories, Inc.)
Stain: Oriole fluorescent gel stain (manufactured by Bio-Rad Laboratories, Inc.)
Analyzer: Imaging system (manufactured by Bio-Rad Laboratories, Inc.)

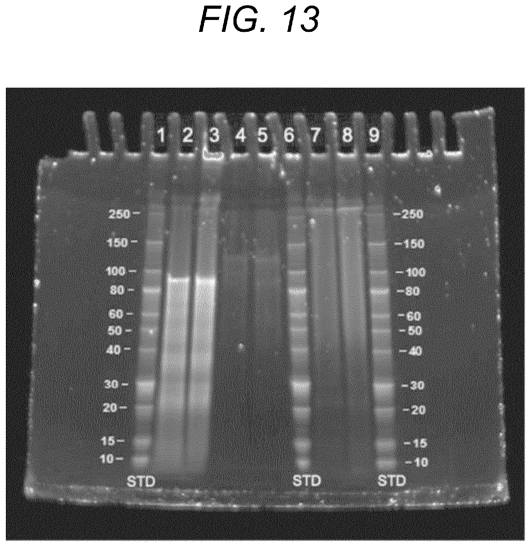

The results are shown in Fig. 13. The lanes are, from the left, a standard (protein ladder), PRT966, acetoacetylated PRT966, PRT587, acetoacetylated PRT587, a standard (protein ladder), silk, acetoacetylated silk, and a standard (protein ladder).

In the lanes 2 and 3, a main band was observed around 100 kDa which is the theoretical molecular weight of the PRT966. In the lane 3 (acetoacetylated PRT966), stronger fluorescence was observed at the top of the gel compared to the lane 2 (PRT966). This was presumed to be because the protein did not migrate due to increase in its hydrophobicity under the above conditions, and thus remained at the initial addition position.

When the lanes 4 and 5 and the lanes 7 and 8 were compared with each other, no band of decomposition products was observed in the region of 100 kDa or less. From this result, it was confirmed that acetoacetylation proceeded without decomposing the protein also in the PRT587 and silk.

### 4. Addition of nucleophile to acetoacetylated protein

### (a) Addition of sodium 5-(2-aminoethylamino)-1-naphthalenesulfonate

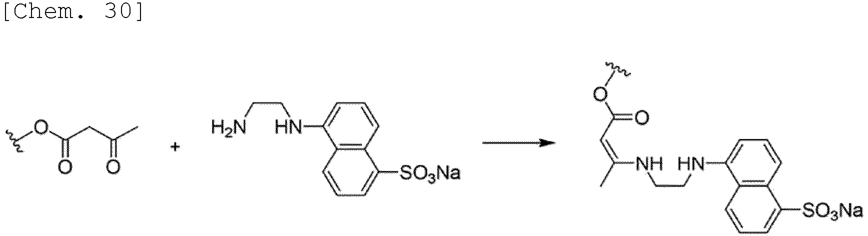

The acetoacetylated PRT966 (49 mg) was weighed in a glass container, DMSO (2.48 mL) was added thereto, and the mixture was heated to 90°C for dissolution. The temperature of this solution was returned to 25°C, and sodium 5-(2-aminoethylamino)-1-naphthalenesulfonate hydrate (1.37 mg, manufactured by Tokyo Chemical Industry Co., Ltd.) was added thereto, thus obtaining a reaction solution. Thereafter, the reaction solution was shielded from light and stirred at 25°C for 20 hours. When 20 hours had elapsed, ethyl acetate (3 mL) was added to the reaction solution, and the mixture was allowed to stand for 2 hours to precipitate a product. The precipitate was collected by filtration, washed sequentially with ethyl acetate, methanol, water, and acetone (10 mL each), and dried under reduced pressure to obtain an amine adduct 1 as a powder. Unreacted products were removed by a washing operation. For comparison, a similar experiment was performed using PRT966 instead of the acetoacetylated PRT966.

### <Fluorescence analysis>

The amine adduct 1 (10 mg) was weighed in a glass container, DMSO (2 mL) was added thereto, and the mixture was heated to 90°C for dissolution. The obtained solution was diluted 200 times, then the diluted solution (200 µL) was placed onto a plate, and subjected to fluorescence analysis under conditions of an excitation wavelength of 336 nm and a fluorescence wavelength of 490 nm.

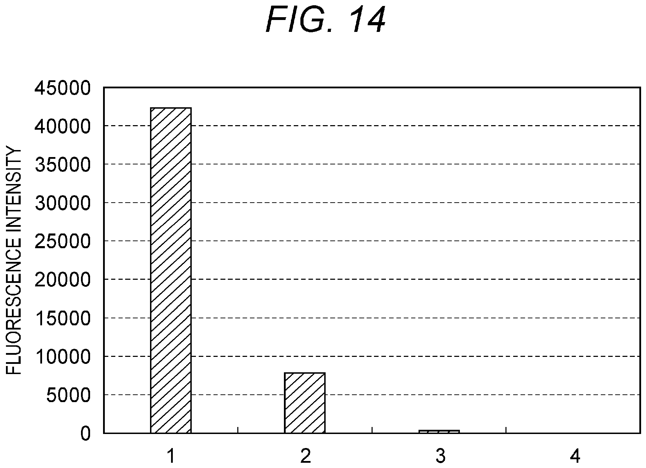

The results are shown in Fig. 14. In Fig. 14, the reference numeral 1 represents the fluorescence intensity of a product (amine adduct 1) obtained by subjecting the acetoacetylated PRT966 to a reaction under the above reaction conditions, the reference numeral 2 represents the fluorescence intensity of a product obtained by subjecting the PRT966 to a reaction under the above reaction conditions, the reference numeral 3 represents the fluorescence intensity of the acetoacetylated PRT966, and the reference numeral 4 represents the fluorescence intensity of the DMSO. In the amine adduct 1, fluorescence was observed, but in the PRT966 and the acetoacetylated PRT966 subjected to the above reaction conditions, fluorescence was hardly observed. Comparison with a calibration curve prepared using sodium 5-(2-aminoethylamino)-1-naphthalenesulfonate showed that five molecules of the nucleophile were added to one molecule of the acetoacetylated PRT966.

### (b) Addition of benzylamine

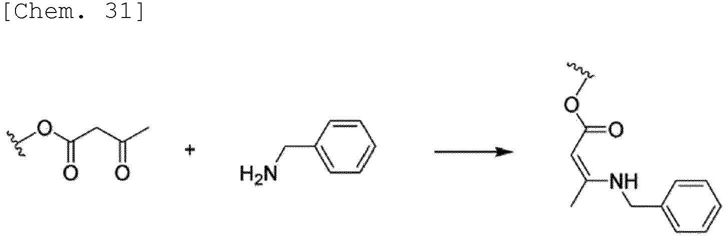

The acetoacetylated PRT966 (413 mg) was weighed in a glass container, DMSO (14.7 mL) was added thereto, and then the mixture was heated to 90°C for dissolution. The temperature of this solution was returned to 60°C, and benzylamine (88.5 µL) and acetic acid (69.5 uL) were added thereto, then the reaction solution was stirred at 60°C for 3 hours. When 3 hours had elapsed, ethyl acetate (45 mL) was added to the reaction solution, and the mixture was allowed to stand for 2 hours to precipitate a product. The precipitate was collected by filtration, washed sequentially with ethyl acetate and acetone (150 mL each), and dried under reduced pressure to obtain an amine adduct 2 as a powder. Unreacted products were removed by a washing operation.

### <¹H-NMR analysis>

In a glass container, 50 mg of the amine adduct 2 or the corresponding acetoacetylated PRT966 was placed, DMSO-d₆ (about 700 µL, manufactured by Eurisotop) was added thereto, and the mixture was heated to 90°C for dissolution. The obtained solution was transferred to an NMR measurement tube (manufactured by FUJIFILM Wako Pure Chemical Corporation), and subjected to 1H-NMR measurement.

### [Measurement conditions]

Apparatus name: JNM-ECZ400R (manufactured by JEOL Ltd.)
Frequency: 400 MHz
Solvent: DMSO-d₆

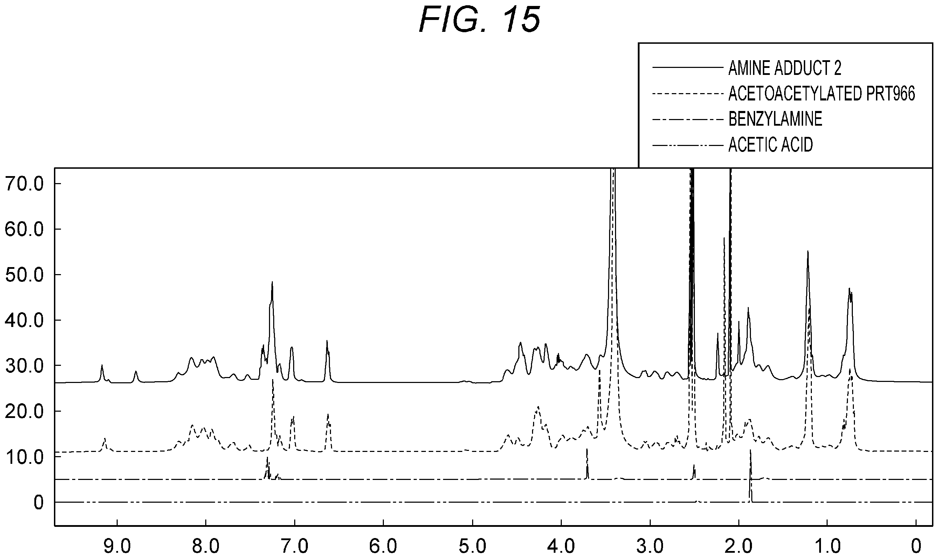

As shown in Fig. 15, in the 1H-NMR spectrum of the amine adduct 2, a peak derived from the terminal methyl group (2.3 ppm) and a peak derived from the NH proton of enamine (8.8 ppm) were observed. In addition, as compared with the unreacted PRT966, a decrease in the peak around 4.9 to 5.1 ppm and a decrease in peaks (3.5 ppm, 2.13 ppm each) derived from the methylene group and the methyl group of the acetoacetyl group were observed.

### (c) Addition of octylamine

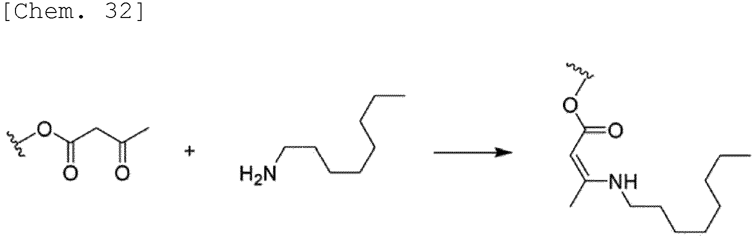

The acetoacetylated PRT966 (80 mg) was weighed in a glass container, DMSO (3.5 mL) was added thereto, and then the mixture was heated to 90°C for dissolution. The temperature of this solution was returned to 60°C, octylamine (25.9 µL) and acetic acid (8.9 µL) were added thereto, and then the reaction solution was stirred at 60°C for 3 hours. When 3 hours had elapsed, acetone (10.5 mL) was added to the reaction solution to precipitate a product. The precipitate was centrifuged, and the solvent was distilled off. This operation was repeated three times in total to perform washing, and the resultant was dried under reduced pressure thereby obtaining an amine adduct 3 as a powder. Unreacted products were removed by a washing operation.

### <¹H-NMR analysis>

In a glass container, 50 mg of the amine adduct 3 or the corresponding acetoacetylated PRT966 was placed, DMSO-d₆ (about 700 µL, manufactured by Eurisotop) was added thereto, and the mixture was heated to 90°C for dissolution. The obtained solution was transferred to an NMR measurement tube (manufactured by FUJIFILM Wako Pure Chemical Corporation), and subjected to ¹H-NMR measurement.

### [Measurement conditions]

Apparatus name: JNM-ECZ400R (manufactured by JEOL Ltd.)
Frequency: 400 MHz
Solvent: DMSO-d6

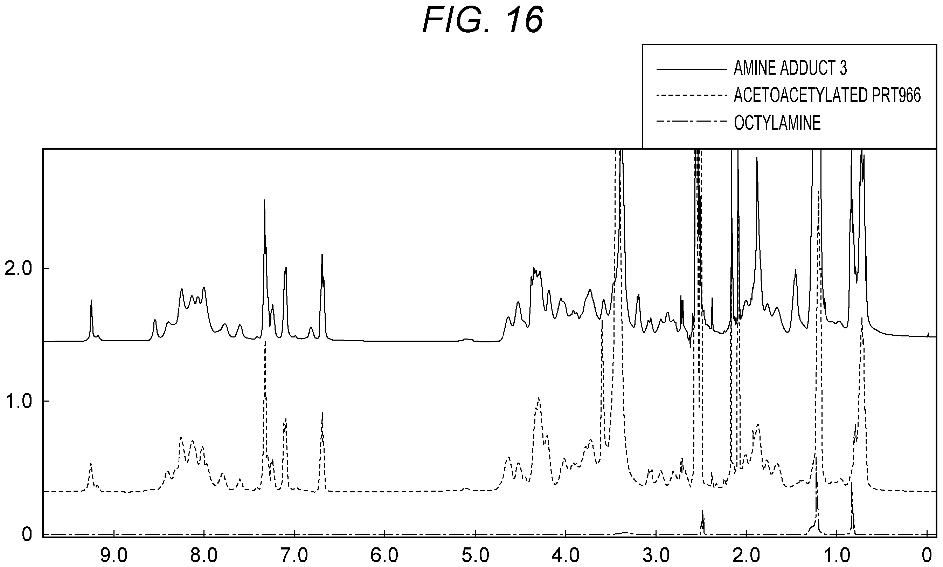

As shown in Fig. 16, in the ¹H-NMR spectrum of the amine adduct 3, a peak derived from the terminal methyl group (1.8 ppm) and a peak derived from the NH proton of enamine (8.4 ppm) were observed, and peaks derived from the added octylamine were observed at 0.8 ppm and 1.4 ppm. In addition, a decrease in peaks (3.5 ppm, 2.13 ppm each) derived from the methylene group and the methyl group of the acetoacetyl group was observed as compared with the unreacted PRT966.

### (d) Addition of furfurylamine

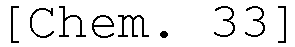

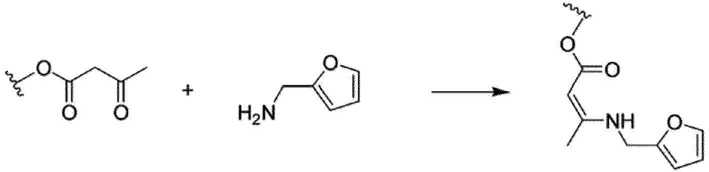

The acetoacetylated PRT966 (84 mg) was weighed in a glass container, DMSO (3.5 mL) was added thereto, and then the mixture was heated to 90°C for dissolution. The temperature of this solution was returned to 60°C, furfurylamine (14.5 µL) and acetic acid (9.4 µL) were added thereto, and then the reaction solution was stirred at 60°C for 3 hours. When 3 hours had elapsed, acetone (10.5 mL) was added to the reaction solution to precipitate a product. The precipitate was centrifuged, and the solvent was distilled off. This operation was repeated three times in total to perform washing, and the resultant was dried under reduced pressure, thereby obtaining an amine adduct 4 as a powder. Unreacted products were removed by a washing operation.

### <¹H-NMR analysis>

In a glass container, 50 mg of the amine adduct 4 or the corresponding acetoacetylated PRT966 was placed, DMSO-d₆ (about 700 µL, manufactured by Eurisotop) was added thereto, and the mixture was heated to 90°C for dissolution. The obtained solution was transferred to an NMR measurement tube (manufactured by FUJIFILM Wako Pure Chemical Corporation), and subjected to NMR measurement.

### [Measurement conditions]

Apparatus name: JNM-ECZ400R (manufactured by JEOL Ltd.)
Frequency: 400 MHz
Solvent: DMSO-d₆

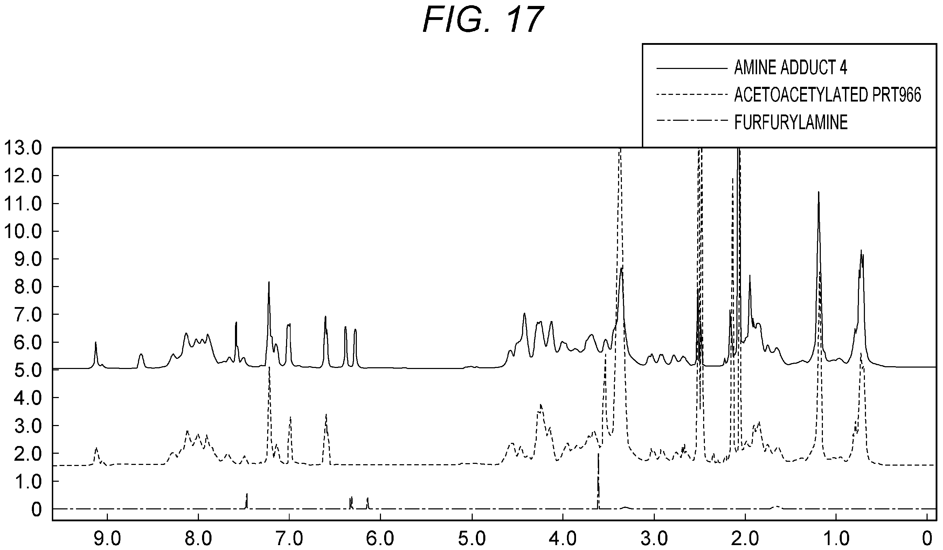

As shown in Fig. 17, in the ¹H-NMR spectrum of the amine adduct 4, a peak derived from the terminal methyl group (1.8 ppm) and a peak derived from the NH proton of enamine (8.6 ppm) were observed, and peaks derived from the added furfurylamine were observed at 6.2 to 6.4 and 7.6 ppm. In addition, a decrease in peaks (3.5 ppm, 2.13 ppm each) derived from the methylene group and the methyl group of the acetoacetyl group was observed.

### 5. Addition of electrophile to acetoacetylated protein

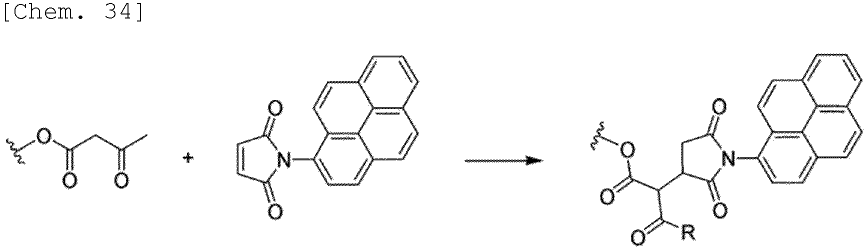

The acetoacetylated PRT966 (49 mg) was weighed in a glass container, DMSO (2.48 mL) was added thereto, and the mixture was heated to 90°C for dissolution. The temperature of this solution was returned to 50°C, and N-(1-pyrenyl)maleimide (1.42 mg, manufactured by Tokyo Chemical Industry Co., Ltd.) and triethylamine (0.66 µL, manufactured by Nacalai Tesque, Inc.) were added thereto, thereby obtaining a reaction solution. Thereafter, the reaction solution was shielded from light and stirred at 50°C for 3 hours. When 3 hours had elapsed, ethyl acetate (3 mL) was added to the reaction solution, and the mixture was allowed to stand for 2 hours to precipitate a product. The precipitate was collected by filtration, washed sequentially with ethyl acetate and acetone (10 mL each), and dried under reduced pressure to obtain a maleimide adduct 1. Unreacted products were removed by a washing operation. For comparison, a similar experiment was performed using PRT966 instead of the acetoacetylated PRT966.

### <Fluorescence analysis>

The maleimide adduct 1 (10 mg) was weighed in a glass container, DMSO (1 mL) was added thereto, and the mixture was heated to 90°C for dissolution. The obtained solution was diluted 20,000 times, then the diluted solution (200 µL) was placed onto a plate, and fluorescence analysis was performed under conditions of an excitation wavelength of 338 nm and a fluorescence wavelength of 375 nm.

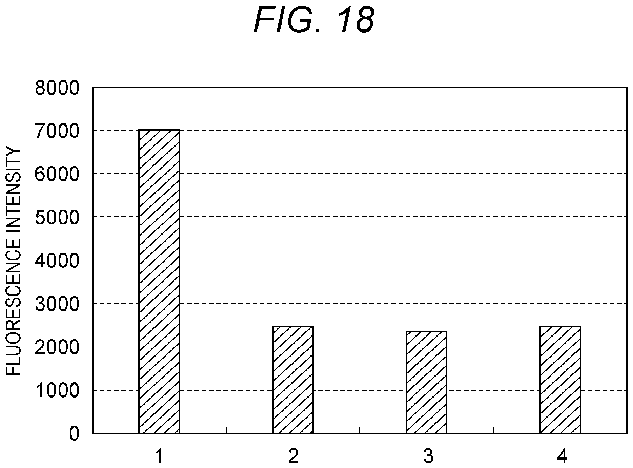

The results are shown in Fig. 18. In Fig. 18, the reference numeral 1 represents the fluorescence intensity of a product (maleimide adduct) obtained by subjecting the acetoacetylated PRT966 to a reaction under the above reaction conditions, the reference numeral 2 represents the fluorescence intensity of a product obtained by subjecting the PRT966 to a reaction under the above reaction conditions, the reference numeral 3 represents the fluorescence intensity of the acetoacetylated PRT966, and the reference numeral 4 represents the fluorescence intensity of only the DMSO. In the maleimide adduct, fluorescence was observed, but in the PRT966 and the acetoacetylated PRT966 subjected to the above reaction conditions, fluorescence was hardly observed.

### 4. Acylation using other β-ketoesters

### (1) Preparation of tert-butyl benzoylacetate

Toluene (100 mL), ethyl benzoylacetate (1.79 mL), tert-butyl alcohol (19.75 mL) and 4-dimethylaminopyridine (381 mg) were added to a glass container equipped with a reflux apparatus to obtain a reaction solution. Thereafter, the reaction solution was stirred for 20 hours under reflux conditions. When 20 hours had elapsed, tert-butyl alcohol (19.75 mL) was added to the reaction solution, and the mixture was further stirred for 20 hours under reflux conditions. The reaction was stopped, and the resulting solution was subjected to purification by silica gel column chromatography (hexane : ethyl acetate = 100 : 1) to obtain tert-butyl benzoylacetate.

### (2) Benzoylacetylation of artificial protein

The artificial protein PRT966 (100 mg) was weighed in a glass container, DMSO (1.7 mL) was added thereto, and then the glass container was heated to 90°C to dissolve the artificial protein, thereby obtaining a reaction solution. The resulting reaction solution was heated to 110°C, tert-butyl benzoylacetate (411 mg) was added to the reaction solution, and the mixture was further reacted for 1 hour. When 1 hour had elapsed, acetone (6 mL) was added to the reaction solution, and the mixture was allowed to stand for 2 hours to precipitate a product. The precipitate was collected by filtration, washed with acetone (20 mL), and dried under reduced pressure to obtain benzoylacetylated PRT966 as a powder. DMSO, unreacted tert-butyl benzoylacetate, and tert-butyl alcohol were removed by the washing operation.

### <¹H-NMR analysis>

In a glass container, 50 mg of the benzoylacetylated PRT966 or unreacted PRT966 (control) was placed, DMSO-d6 (about 700 µL, manufactured by Eurisotop) was added thereto, and the mixture was heated to 90°C for dissolution. The obtained solution was transferred to an NMR measurement tube (manufactured by FUJIFILM Wako Pure Chemical Corporation), and subjected to ¹H-NMR measurement.

### [Measurement conditions]

Apparatus name: JNM-ECZ400R (manufactured by JEOL Ltd.)
Frequency: 400 MHz
Solvent: DMSO-d₆

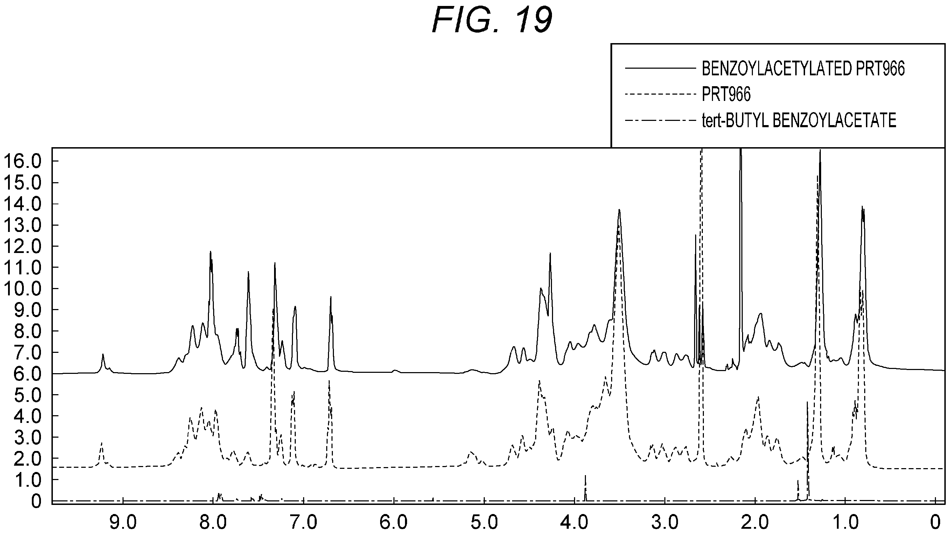

As shown in Fig. 19, in the ¹H-NMR spectrum of the benzoylacetylated PRT966, peaks derived from the benzoylacetyl group (4.1 ppm, 7.5 to 8.0 ppm) were observed. In addition, as compared with the unreacted PRT966, the peak (around 4.9 to 5.1 ppm) derived from the hydroxy group used in the acylation reaction was reduced.

### 7. Coagulation performance in spinning process

### (1) Preparation of DMSO dope solution of artificial protein

The artificial protein PRT966 (1.0 g) was weighed and added to a DMSO (19.0 g) solution in which 4 wt% lithium chloride was dissolved so as to have a final concentration of 20 wt%, and the mixture was stirred at 100°C for 30 minutes to prepare a DMSO dope solution A.

### (2) Preparation of DMSO dope solution of acetoacetylated artificial protein

The artificial protein PRT966 (1.0 g) was weighed and added to a DMSO (19.0 g) solution in which 4 wt% lithium chloride was dissolved so as to have a final concentration of 20 wt%, and the mixture was stirred at 100°C for 30 minutes. Thereafter, the reaction solution was heated to 110°C, tert-butyl acetoacetate (0.5 g or 1.0 g) was added thereto with stirring, and the mixture was stirred for 1 hour. In this way, two DMSO dope solutions B and C having different degrees of acetoacetylation treatment were prepared.

### (3) Evaluation of coagulability of DMSO dope solution in water

Each of the three types of DMSO dope solutions (1.0 mL) obtained in the above (1) or (2) was filled in a syringe equipped with a needle having an inner diameter of 0.47 mm. Water (80 mL) at 75°C was provided in a 100 mL beaker. While the tip of the needle was placed in water, the entire amount of the DMSO dope solution was slowly poured. The state of the material in water was visually observed and evaluated according to the following criteria.

### [Evaluation criteria of coagulability]

1: After discharge from the needle, the material does not become a thread due to fusion of the materials.
2: Fusion immediately after discharge is not observed, but the materials are gradually fused to each other, and do not become a thread.
3: Threads are formed, but the threads are partially fused to each other.
4: Threads are precipitated on the bottom of the beaker and fused with the bottom of the beaker.
5: No fusion is observed.

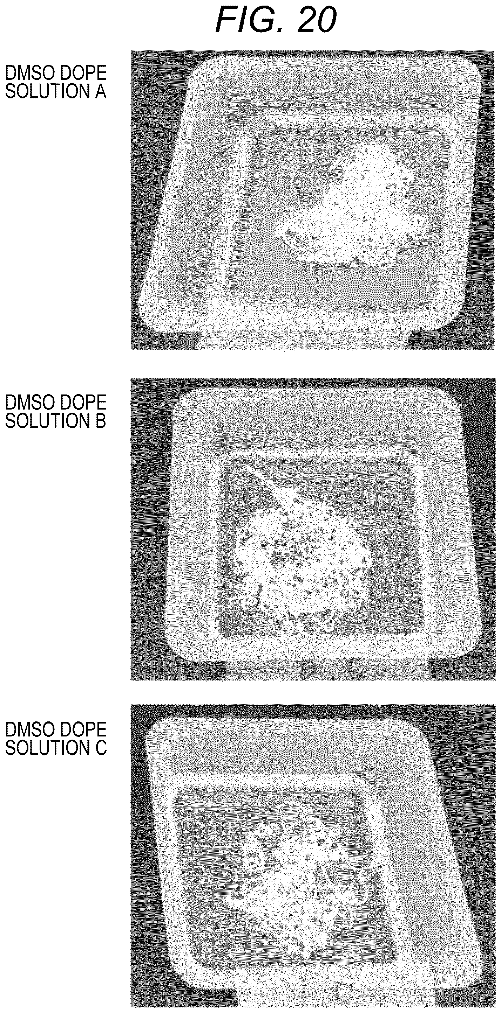

The results are shown in Fig. 20. The DMSO dope solution B was superior in coagulability to the DMSO dope solution A, and the DMSO dope solution C showed further superior coagulability to the DMSO dope solution B. Specifically, the dope solutions A, B, and C were evaluated as 3, 4, and 5, respectively.

### [Sequence Listing]

International application FP22-1190-Esterified protein and method for producing the same JP22041016 20221102----00670185552202460112 Normal 20221102132749202210281124190140-P1AP101--FP-39.xml under the International Patent Cooperation Treaty

## Claims

1. A β-ketoester of a protein containing an amino acid having a hydroxyl group, wherein
the hydroxyl group forms an ester bond with a group represented by Formula (1): 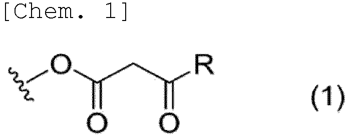
wherein R represents a C₁₋₆ alkyl group or an optionally substituted aryl group.

2. The β-ketoester of the protein according to claim 1, wherein the protein is a hydrophobic protein.

3. The β-ketoester of the protein according to claim 2, wherein the hydrophobic protein includes a protein having an average hydropathy index (HI) of -1 or more.

4. The β-ketoester of the protein according to any one of claims 1 to 3, wherein the protein is an artificial protein.

5. A method for producing the β-ketoester of the protein according to claim 1,
the method comprising a step of mixing the protein containing an amino acid having a hydroxyl group and a compound represented by Formula (2a) or (2b) in a solvent and performing stirring while heating: 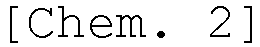 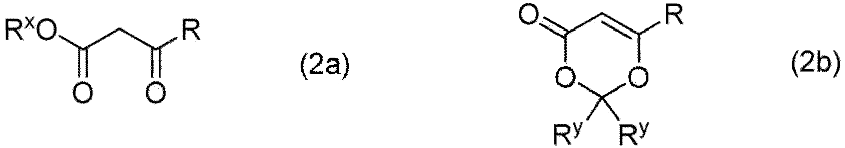
wherein R represents a C₁₋₆ alkyl group or an optionally substituted aryl group, and R^{x} and R^{y} each independently represent a C₁₋₆ alkyl group or an optionally substituted aryl group.

6. The method for producing the β-ketoester of the protein according to claim 5, wherein the protein includes a hydrophobic protein.

7. The method for producing the β-ketoester of the protein according to claim 6, wherein the hydrophobic protein includes a protein having an average hydropathy Index (HI) of -1 or more.

8. The method for producing the β-ketoester of the protein according to any one of claims 5 to 7, wherein the protein includes an artificial protein.

9. A method for producing a chemically modified protein represented by Formula (3), the method comprising:
a step of mixing the β-ketoester of the protein according to claim 1 and a compound represented by Formula (4) in a solvent: 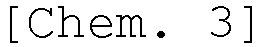 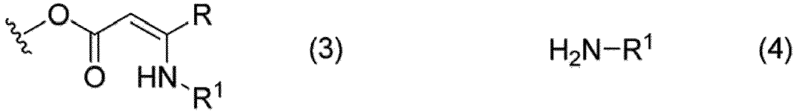
wherein R represents a C₁₋₆ alkyl group or an optionally substituted aryl group, and R¹ represents an organic group.

10. A chemically modified protein having a group represented by Formula (3): 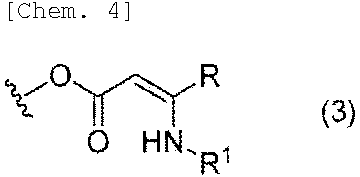 wherein R represents a C₁₋₆ alkyl group or an optionally substituted aryl group, and R¹ represents an organic group.

11. A method for producing a chemically modified protein represented by Formula (5), the method comprising:
a step of mixing the β-ketoester of the protein according to claim 1 and a compound represented by Formula (6) in a solvent: 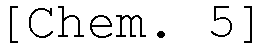 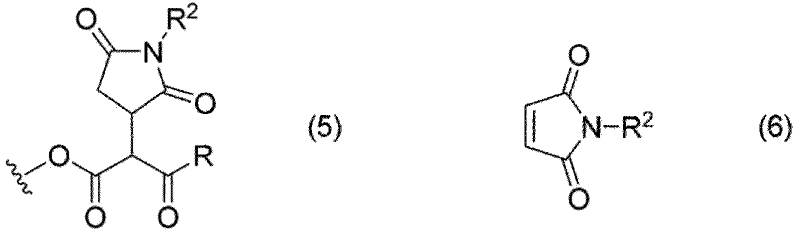
wherein R represents a C₁₋₆ alkyl group or a phenyl group, and R² represents an organic group.

12. A chemically modified protein having a group represented by Formula (5): 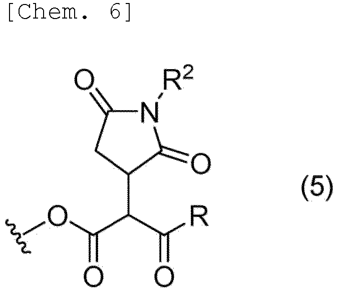 wherein R represents a C₁₋₆ alkyl group or an optionally substituted aryl group, and R² represents an organic group.
